# EUROPEAN PATENT APPLICATION

(11) **EP 0 816 362 A1**
(43) Date of publication of application: **07.01.1998**
(21) Application number: 96906932.7
(22) Date of filing: 22.03.1996
(51) Int. Cl.: C07D 417/04, C07D 417/06, C07D 417/14, A61K 31/425, A61K 31/445, A61K 31/495

(54) **THIAZOLE DERIVATIVES**

(30) Priority: 22.03.1995 JP 62326/95; 22.03.1995 JP 62327/95; 22.03.1995 JP 62328/95; 22.03.1995 JP 62329/95; 07.11.1995 JP 287741/95; 07.11.1995 JP 287742/95; 07.11.1995 JP 287743/95; 07.11.1995 JP 287744/95
(71) Applicant: TAISHO PHARMACEUTICAL CO. LTD, Tokyo 171 (JP); Nihon Nohyaku Co., Ltd., Tokyo 103 (JP)
(72) Inventor: NAKAZATO, Atsuro, Toshima-ku, Tokyo 171 (JP); KUMAGAI, Toshihito, Toshima-ku, Tokyo 171 (JP); CHAKI, Shigeyuki, Toshima-ku, Tokyo 171 (JP); TOMISAWA, Kazuyuki, Toshima-ku, Tokyo 171 (JP); NAGAMINE, Masashi, Res. Center of, Kawachinagano-shi, Osaka 586 (JP); GOTOH, Makoto, Res. Center of, Kawachinagano-shi, Osaka 586 (JP); YOSHIDA, Masanori, Res. Center of, Kawachinagano-shi, Osaka 586 (JP)
(74) Representative: Weisert, Annekäte, Dipl.-Ing. Dr.-Ing.
(86) International application number: JP9600763
(87) International publication number: WO9629330

(57) **Abstract**

The present invention relates to a thiazole derivative represented by formula (I) 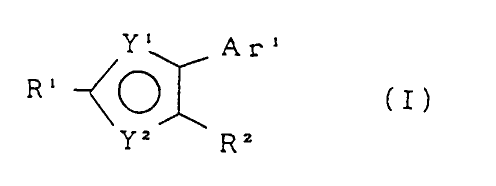 wherein Ar¹ represents a substituted or unsubstituted phenyl or thienyl group, Y¹ and Y² are different and each represents a nitrogen atom or a sulfur atom, R¹ represents a hydrogen atom, a C₁ to C₅ alkyl group, a phenyl group, or an amino group unsubstituted or mono- or di-substituted with a C₁ to C₅ alkyl group, and R² represents a substituted or unsubstituted nitrogen heterocycle group, or a pharmaceutically acceptable salt thereof. The compound of the present invention are a dopamine D₄ receptor antagonist which has an antipsychotic effect without causing any extrapyramidal disorder.

## Description

### Technical Field

The present invention relates to thiazole derivatives having an antipsychotic effect.

### Background Art

The antipsychotic drug is used for treatment of schizophrenia as well as treatment of problematic behaviors in cerebrovascular disease and senile dementia (offensive behaviors, mental excitement, wandering, delirium etc.). However, a dopamine D₂ receptor antagonist which is a conventional antipsychotic drug causes strong extrapyramidal disorder as side effects, and this is a great problem.

On the other hand, the structure and properties of a recently found dopamine D₄ receptor are similar to those of dopamine D₂ receptor, and the major difference therebetween is their distribution in the brain. The dopamine D₄ receptor is distributed at higher concentration in the frontal lobe of the cerebral cortex involved in the onset of schizophrenia while at less concentration in striatum involved in developing extrapyramidal disorder. Therefore, it is highly possible that a dopamine D₄ receptor antagonist, unlike the dopamine D₂ receptor antagonist, serves as a novel drug for treating schizophrenia without causing extrapyramidal disorder as side effects (Nature, 350, 610-614 (1991); Nature, 358, 109 (1992); Nature, 365, 393 (1993); and Nature, 365, 441-445 (1993)).

This kind of compound includes clozapine. It is reported that the affinity of clozapine is higher for the dopamine D₄ receptor than for the dopamine D₂ receptor (Nature, 350, 610-614 (1991)). Further, it is reported in clinical experiments that clozapine, unlike the dopamine D₂ receptor antagonist, is effective against drug-resistant schizophrenia and negative symptoms and it causes less extrapyramidal disorder (Arch. Gen. Psych., 45, 789-796 (1988)). However, clozapine causes a blood disorder called agranulocytosis, and a case of death is also reported (Summary and Clinical Data, Sandoz, Canada Inc. (1990)), and this is a great problem.

Therefore, the usefulness of the dopamine D₄ receptor antagonist without such side effects is high as a drug for treating schizophrenia etc. by which the extrapyramidal disorder is not or hardly caused.

### Disclosure of the Invention

An object of the present invention is to provide a dopamine D₄ receptor antagonist which has an antipsychotic effect without causing extrapyramidal disorder.

As a result of their extensive study on thiazole derivatives, the present inventors found novel thiazole derivatives having a high affinity for dopamine D₄ receptor to complete the present invention.

Hereinafter, the present invention is described.

The present invention comprises the following inventions:
(A) A thiazole derivative represented by formula (I) 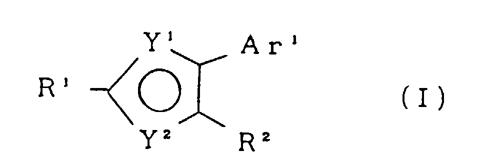 wherein Ar¹ represents a substituted or unsubstituted phenyl group or a thienyl group, Y¹ and Y² are different and each represents a nitrogen atom or a sulfur atom, R¹ represents a hydrogen atom, a C₁ to C₅ alkyl group, a phenyl group, or an amino group unsubstituted or mono- or di-substituted with a C₁ to C₅ alkyl group;
   R² represents a group represented by formula (i): 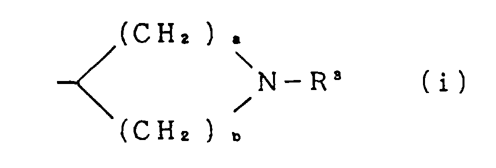 wherein a is an integer of 1 to 3, b is 2 or 3;
   R³ represents:
   (1) a hydrogen atom, a substituted or unsubstituted C₁ to C₇ alkyl group, a substituted or unsubstituted C₃ to C₇ alkenyl group, a C₃ to C₇ alkynyl group or a C₂ to C₇ alkoxycarbonyl group,
   (2) a group represented by the formula: 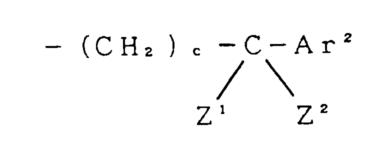 wherein c is an integer of 1 to 5, Ar² represents a substituted or unsubstituted phenyl group or a thienyl group, Z¹ and Z² are the same or different and each represents a C₁ to C₅ alkoxy group or they are combined to form an oxo group or a C₂ to C₃ alkylenedioxy group,
   (3) a group represented by the formula: 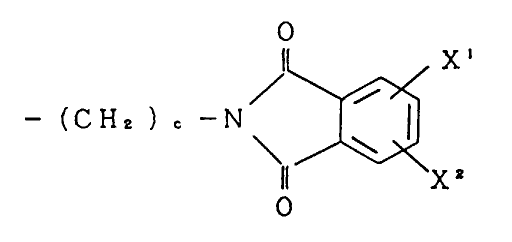 wherein c has the same meaning as defined above, X¹ and X² are the same or different and each represents a hydrogen atom, a halogen atom, a C₁ to C₅ alkoxy group or a hydroxyl group;
   (4) a group represented by the formula: 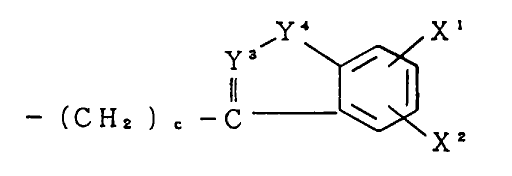 wherein c, X¹ and X² have the same meaning as defined above, Y³ represents N or CH, Y⁴ represents an oxygen atom, sulfur atom or NH; or
   (5) a group represented by the formula: 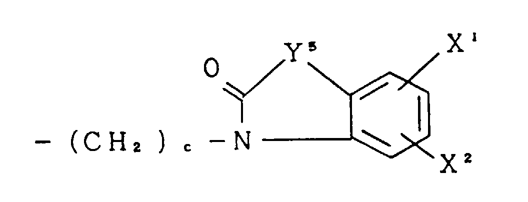 wherein c, X¹ and X² have the same meaning as defined above, and Y⁵ represents CH₂-CH₂, CH=CH, CH₂ or NH;
      a group represented by formula (ii): 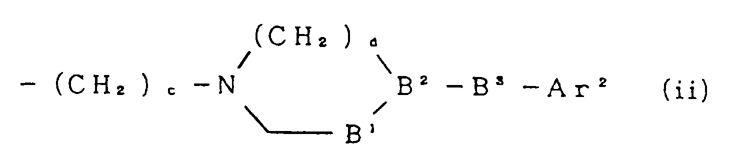 wherein c and Ar² have the same meaning as defined above, d is 1 or 2, B¹-B² represents CH₂-CH, CH=C or CH₂-N, B³ is a group represented by the formula: 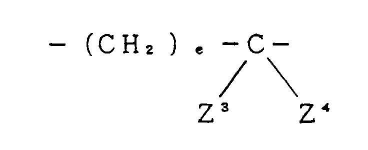 wherein e is 0 or 1, Z³ and Z⁴ both represent a hydrogen atom or are the same or different and each represents a C₁ to C₅ alkoxy group or they are combined to form an oxo group, a methylene group or a C₂ to C₃ alkylenedioxy group, or
      a group represented by formula (iii): 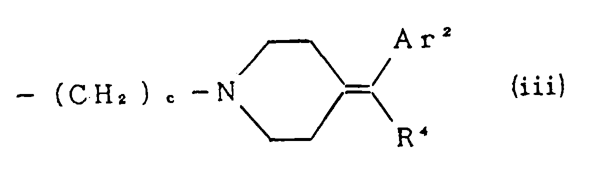 wherein c and Ar² have the same meaning as defined above, R⁴ represents a hydrogen atom, a C₁ to C₅ alkyl group or a substituted or unsubstituted phenyl group, or a pharmaceutically acceptable salt thereof.
(B) The compound of formula (I), wherein Y¹ is a nitrogen atom, and Y² is a sulfur atom.
(C) A thiazole derivative represented by formula (I-i-1): 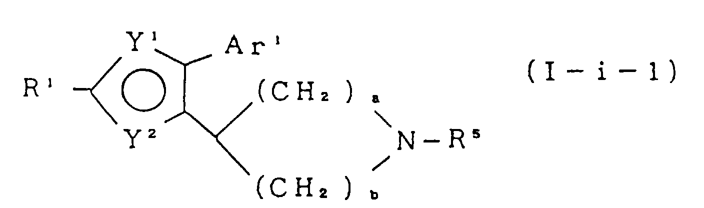 wherein Ar¹, Y¹, Y², R¹, a and b have the same meaning as in (A) above; R⁵ is a hydrogen atom, a substituted or unsubstituted C₁ to C₇ alkyl group, a substituted or unsubstituted C₃ to C₇ alkenyl group, a C₃ to C₇ alkynyl group or a C₂ to C₇ alkoxycarbonyl group, or a pharmaceutically acceptable salt thereof.
(D) The compound of formula (I-i-1), wherein Ar¹ is a phenyl group substituted with 1 or 2 groups selected from a halogen atom and a C₁ to C₅ alkoxy group, or a phenyl group, Y¹ is a nitrogen atom, Y² is a sulfur atom, R¹ is a hydrogen atom, a C₁ to C₅ alkyl group, an amino group or a C₁ to C₅ monoalkylamino group, R⁵ is a C₁ to C₆ alkyl group substituted at the terminal with 1 or 2 groups selected from "a phenyl group substituted with 1 or 2 groups selected from a halogen atom and a C₁ to C₅ alkoxy group, and a phenyl group", a C₃ to C₅ alkenyl group substituted at the terminal with 1 or 2 groups selected from "a phenyl group substituted with 1 or 2 groups selected from a halogen atom and a C₁ to C₅ alkoxy group, and a phenyl group", and a C₂ to C₇ alkoxycarbonyl group.
(E) A thiazole derivative represented by formula (I-i-2): 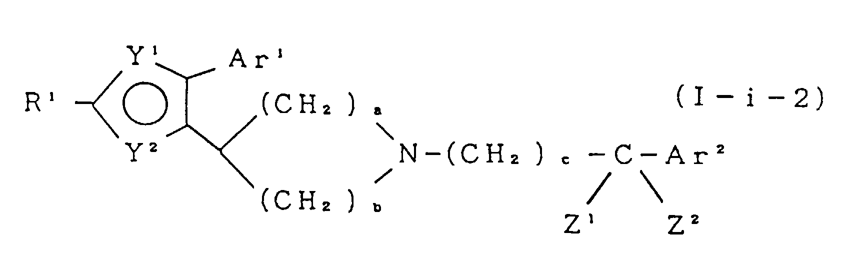 wherein Ar¹, Ar², Y¹, Y², R¹, a, b, c, Z¹ and Z² have the same meaning as in (A) above, or a pharmaceutically acceptable salt thereof.
(F) The compound of formula (I-i-2), wherein Ar¹ and Ar² are the same or different and each represents a phenyl group unsubstituted or substituted with 1 or 2 halogen atoms, Y¹ is a nitrogen atom, Y² is a sulfur atom, R¹ is a hydrogen atom, a C₁ to C₅ alkyl group, an amino group or a C₁ to C₅ monoalkylamino group, and Z¹ and Z² are combined to form an oxo group.
(G) A thiazole derivative represented by formula (I-i-4): 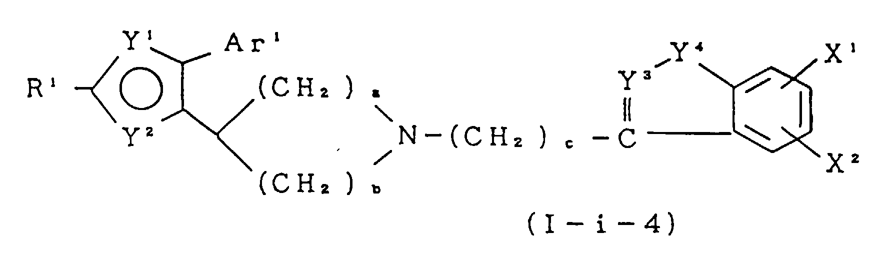 wherein Ar¹, Y¹, Y², Y³, Y⁴, R¹, a, b, c, X¹ and X² have the same meaning as in (A) above, or pharmaceutically acceptable salts thereof.
(H) The compound of formula (I-i-4), wherein Ar¹ is a phenyl group unsubstituted or substituted with 1 or 2 halogen atoms, Y¹ is a nitrogen atom, Y² is a sulfur atom, and R¹ is a hydrogen atom, a C₁ to C₅ alkyl group, an amino group or a C₁ to C₅ monoalkylamino group.
(I) A thiazole derivative represented by formula (I-ii): 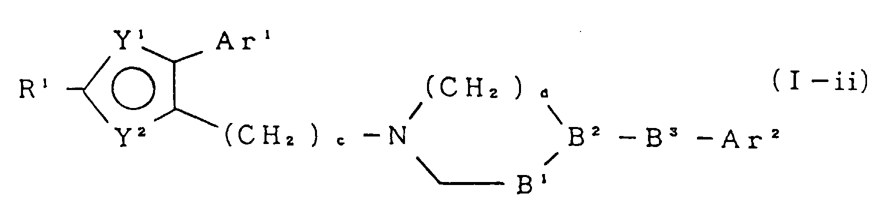 wherein Ar¹, Ar², Y¹, Y², R¹, c, d, B¹, B² and B³ have the same meaning as in (A) above, or a pharmaceutically acceptable salt thereof.
(J) The compound of formula (I-ii), wherein Ar¹ and Ar² are the same or different and each represents a phenyl group substituted with 1 or 2 groups selected from a halogen atom, a C₁ to C₅ alkyl group, a C₁ to C₅ alkoxy group, a hydroxyl group and a trifluoromethyl group, or a phenyl group, Y¹ is a nitrogen atom, Y² is a sulfur atom, and R¹ is a hydrogen atom, a C₁ to C₅ alkyl group, an amino group or a C₁ to C₅ monoalkylamino group.
(K) The compound of formula (I-ii), wherein Ar¹ and Ar² are the same or different and each represents a phenyl group substituted with 1 or 2 groups selected from a halogen atom and a C₁ to C₅ alkoxy group, or a phenyl group, Y¹ is a nitrogen atom, Y² is a sulfur atom, c is 2 or 3, B¹-B² is CH₂-CH, B³ is CO, and R¹ is a hydrogen atom, a methyl group, an amino group or a methylamino group.
(L) A thiazole derivative represented by formula (I-iii): 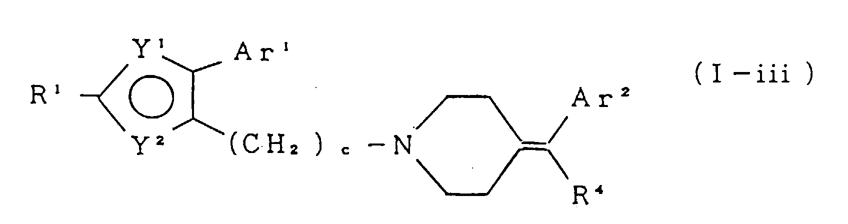 wherein Ar¹, Ar², Y¹, Y², R¹, R⁴ and c have the same meaning as in (A) above, or a pharmaceutically acceptable salt thereof.
(M) The compound of formula (I-iii), wherein Ar¹ and Ar² are the same or different and each represents a phenyl group substituted with 1 or 2 groups selected from a halogen atom, a C₁ to C₅ alkyl group, a C₁ to C₅ alkoxy group, a hydroxyl group and a trifluoromethyl group, or a phenyl group, Y¹ is a nitrogen atom, Y² is a sulfur atom, and R¹ is a hydrogen atom, a C₁ to C₅ alkyl group, an amino group or a C₁ to C₅ monoalkylamino group.
(N) The compound of formula (I-iii), wherein Ar¹ and Ar² are the same or different and each represents a phenyl group substituted with 1 or 2 groups selected from a halogen atom and a C₁ to C₅ alkoxy group, or a phenyl group, Y¹ is a nitrogen atom, Y² is a sulfur atom, c is 2 or 3, R¹ is a hydrogen atom, a methyl group, an amino group or a methylamino group, and R⁴ is a hydrogen atom, a C₁ to C₅ alkyl group, a phenyl group substituted with a halogen atom, or a phenyl group.

In the present invention, the substituted phenyl group represented by Ar¹, Ar² or R⁴ includes e.g. a phenyl group substituted with 1 or 2 groups selected from a halogen atom, a C₁ to C₅ alkyl group, a C₁ to C₅ alkoxy group, a hydroxyl group, and a trifluoromethyl group.

The substituted C₁ to C₇ alkyl group or C₃ to C₇ alkenyl group represented by R³ includes e.g. a C₁ to C₇ alkyl group or a C₃ to C₇ alkenyl group substituted preferably at the terminal with 1 or 2 groups selected from a phenyl group, a naphthyl group and an indolyl group which are unsubstituted or substituted with 1 or 2 groups selected from a hydroxyl group, a C₁ to C₅ alkoxy group and a halogen atom.

The halogen atom in the present invention includes a fluorine atom, chlorine atom, bromine atom or iodine atom, preferably fluorine atom.

The C₁ to C₅ alkyl group and C₁ to C₇ alkyl group is a straight-chain, branched chain or cyclic alkyl group, or an alkyl group substituted with a cyclic alkyl group, and examples include methyl group, ethyl group, propyl group, isopropyl group, butyl group, isobutyl group, cyclopropylmethyl group, pentyl group, isopentyl group, hexyl group, cyclopentylmethyl group, heptyl group, cyclohexylmethyl group etc. The C₃ to C₇ alkenyl group is a straight-chain or branched chain alkenyl group, and examples include 2-propen-1-yl group, 3-methyl-2-buten-1-yl group etc. The C₃ to C₇ alkynyl group is a straight-chain or branched chain alkynyl group, and examples include 2-propyn-1-yl group etc.

The C₁ to C₅ alkoxy group is a straight-chain or branched chain alkoxy group, and examples include methoxy group, ethoxy group, propoxy group, isopropoxy group, butoxy group, isobutoxy group, t-butoxy group, pentoxy group, 3-methylbutoxy group etc.

The C₂ to C₇ alkoxycarbonyl group is a straight-chain or branched chain alkoxycarbonyl group, and examples include methoxycarbonyl group, ethoxycarbonyl group, propoxycarbonyl group, isopropoxycarbonyl group, butoxycarbonyl group, isobutoxycarbonyl group, t-butoxycarbonyl group, pentoxycarbonyl group, 3-methylbutoxycarbonyl group etc.

The amino group which is mono- or di-substituted with C₁ to C₅ alkyl groups includes e.g. methylamino group, dimethylamino group, ethylamino group, diethylamino group etc.

The phenyl group substituted with 1 or 2 groups selected from a halogen atom, a C₁ to C₅ alkyl group, a C₁ to C₅ alkoxy group, hydroxyl group and trifluoromethyl group includes e.g. 2-fluorophenyl group, 3-fluorophenyl group, 4-fluorophenyl group, 4-chlorophenyl group, 4-bromophenyl group, 2,4-difluorophenyl group, 3,4-dichlorophenyl group, 3-bromo-4-methoxyphenyl group, 4-methylphenyl group, 3,4-dimethylphenyl group, 4-isopropylphenyl group, 4-methoxyphenyl group, 3,4-dimethoxyphenyl group, 4-isopropoxyphenyl group, 4-hydroxyphenyl group, 2-trifluroromethylphenyl group, 3-trifluoromethylphenyl group, 4-trifluoromethylphenyl group etc.

The C₁ to C₇ alkyl group substituted at the terminal with 1 or 2 groups selected from a phenyl group, a naphthyl group and an indolyl group unsubstituted or substituted with 1 or 2 groups selected from a hydroxyl group, C₁ to C₅ alkoxy group and halogen atom includes e.g. benzyl group, 4-fluorobenzyl group, 2-phenylethyl group, 2-(4-fluorophenyl)ethyl group, 2-(3,4-dimethoxyphenyl)ethyl group, 2-(indol-3-yl)ethyl group, 3-phenylpropyl group, 3-(4-fluorophenyl)propyl group, 4-phenylbutyl group, 4-(4-chlorophenyl) butyl group, 4-(4-fluorophenyl)butyl group, 4,4-bis(4-fluorophenyl) butyl group, 5-(3-bromophenyl)pentyl group etc.

The C₃ to C₇ alkenyl group whose terminal is substituted with 1 or 2 groups selected from a phenyl group, naphthyl group and indolyl group unsubstituted or substituted with 1 or 2 groups selected from a hydroxyl group, a C₁ to C₅ alkoxy group and halogen atom includes e.g. (2E)-3-phenyl-2-propen-1-yl group, (3E)-4-(4-fluorophenyl)-3-buten-1-yl group, 4,4-bis(4-fluorophenyl)-3-buten-1-yl group etc.

The pharmaceutically acceptable salt in the present invention includes e.g. salts with mineral acids such as sulfuric acid, hydrochloric acid, phosphoric acid etc. and salts with organic acids such as acetic acid, oxalic acid, lactic acid, tartaric acid, fumaric acid, maleic acid, trifluoroacetic acid, methanesulfonic acid, pamoic acid, decanoic acid, enanthic acid etc.

The compounds of formula (I) also include optically active derivatives if any due to the presence of an asymmetric carbon.

The compounds of formula (I) can be produced in the following manners.

In the following reaction formulae, Ar¹, Ar², B¹, B², R¹, R², R³, R⁴, X¹, X², Y¹, Y², Y³, Y⁴, Y⁵, Z¹, Z², Z³, Z⁴, a, b, c, d and e have the same meaning as defined in formula (I) above.
M represents a hydrogen atom or an alkali metal atom such as lithium, sodium or potassium;
R⁴ represents a C₁ to C₆ alkyl group or a benzyl group;
R⁷ represents R³ excluding a hydrogen atom and a C₂ to C₇ alkoxycarbonyl group;
R⁸ and R⁹ are the same or different and each represents a C₁ to C₅ alkyl group or they are combined to form (CH₂)₂ or (CH₂)₃;
R¹⁰ represents a C₁ to C₅ alkyl group, a C₂ to C₆ alkoxycarbonyl group, a benzyloxycarbonyl group, a C₂ to C₇ acyl group, C₁ to C₇ alkanesulfonyl group or a toluenesulfonyl group;
X³ represents a chlorine atom, a bromine atom, an iodine atom, a C₁ to C₇ alkanesulfonyloxy group or a toluenesulfonyloxy group;
X⁴ represents a bromine atom or an iodine atom;
X⁵ represents a chlorine atom, a bromine atom or an iodine atom;
X⁶ and X⁷ are the same or different and each represents a C₁ to C₅ alkoxy group or are different and each represents a hydrogen atom or a C₁ to C₅ alkoxy group;
X⁸ represents a hydroxyl group when X¹ represents an alkoxy group, and X⁸ represents X¹ when X¹ represents a group other than alkoxy group;
X⁹ represents a hydroxyl group when X² represents an alkoxy group, and X⁹ represents X² when X² represents a group other than alkoxy group;
X¹⁰ and X¹¹ are the same and each represents a hydroxyl group or are different and each represents a hydroxyl group and a hydrogen atom;
Y⁶ and Y⁷ are different and each represents a carbonyl group or a methylene group;
Z⁵ and Z⁶ both represent a hydrogen atom or are combined to form an oxo group or a methylene group;
f is 0 or an integer of 1 to 6.

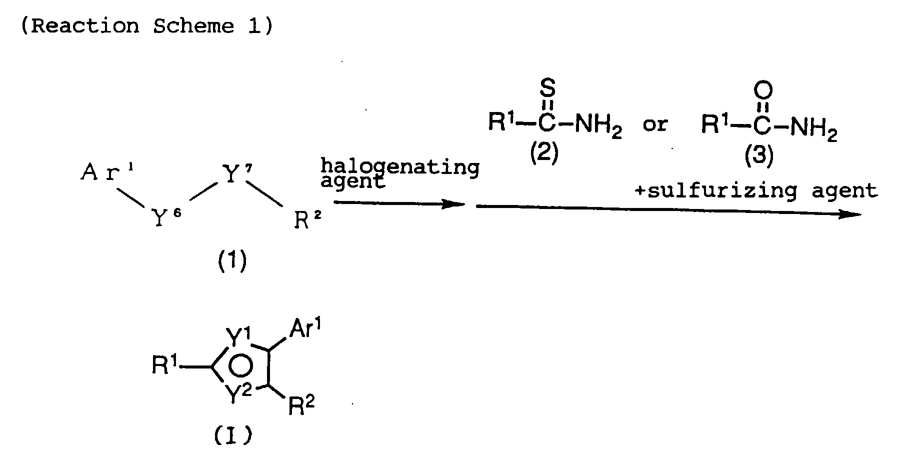

Ketone derivative (1) (Chem. Pharm. Bull., 25, 1911 (1977), J. Med. Chem., 35, 4334 (1992)) is halogenated with a halogenating agent in an inert solvent and then reacted in an inert solvent with a thiourea derivative or thioamide derivative represented by formula (2), or reacted with an urea derivative or amide derivative represented by formula (3) along with a sulfurizing agent, whereby the compound of the present invention represented by formula (I) can be obtained.

The inert solvent mentioned here includes an organic carboxylic acid such as acetic acid, organic halogen compounds such as chloroform, carbon tetrachloride etc., alcohols such as ethanol, isopropanol etc., ethers such as diethyl ether, tetrahydrofuran, dioxane etc., hydrocarbons such as benzene, toluene etc., and water as well as mixed solvents thereof.

The halogenating agent includes chlorine, bromine, iodine, N-chlorosuccinimide, N-bromosuccinimide, etc.

The sulfurizing agent includes e.g. phosphorous pentasulfide, Lawesson's reagent etc.

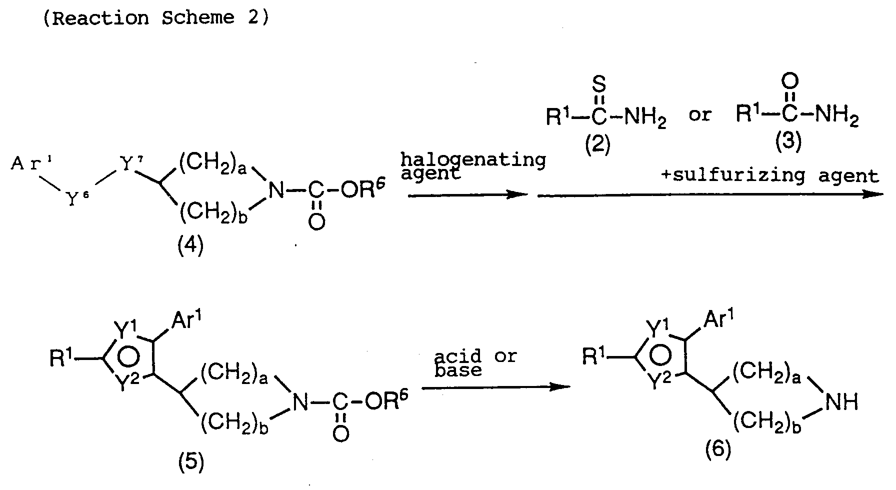

N-alkoxycarbonyl derivative (4) is halogenated in the same manner as described above and then reacted with a thiourea derivative or thioamide derivative represented by formula (2), or reacted with an urea derivative or amide derivative represented by formula (3) along with a sulfurizing agent, whereby compound (5) of the present invention is obtained.

Further, compound (5) is hydrolyzed with an acid or a base in an inert solvent whereby compound (6) of the present invention is obtained.

The acid mentioned here includes e.g. inorganic acids such as hydrochloric acid, hydrobromic acid, sulfuric acid etc., and organic acids such as trifluoroacetic acid etc., and the base includes e.g. inorganic bases such as sodium hydroxide, potassium hydroxide, barium hydroxide etc.

The inert solvent includes e.g. organic carboxylic acids such as acetic acid, organic halogen compounds such as chloroform, dichloromethane etc., alcohols such as ethanol, isopropanol etc., ethers such as tetrahydrofuran, dioxane etc., hydrocarbons such as toluene etc., ketone compounds such as acetone, methyl ethyl ketone etc., and water as well as mixed solvents thereof.

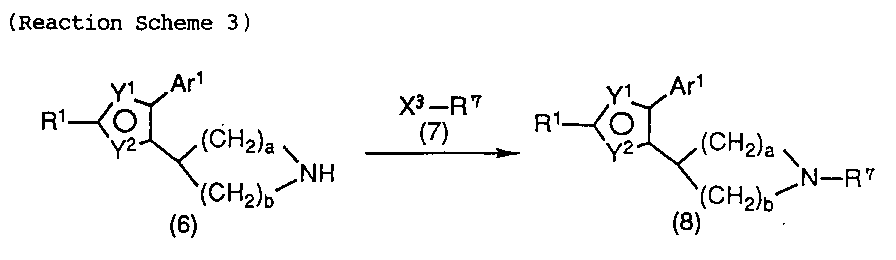

NH derivative (6) obtained by the above reaction can also be reacted with compound (7) in the presence of a base in an inert solvent to obtain compound (8) of the present invention.

The base mentioned here includes e.g. organic amines such as triethylamine, diisopropylethylamine, pyridine etc., alcoholates such as sodium ethoxide etc., alkali metal amides such as sodium amide etc., inorganic bases such as sodium hydrogen carbonate, sodium carbonate, potassium carbonate, sodium hydroxide, sodium hydride etc. and organic salts such as sodium acetate etc.

The inert solvent includes e.g. organic carboxylic acids such as acetic acid, organic halogen compounds such as chloroform, carbon tetrachloride etc., alcohols such as ethanol, isopropanol etc., ethers such as tetrahydrofuran, dioxane etc., hydrocarbons such as benzene, toluene etc., ketone compounds such as acetone, methyl ethyl ketone etc., N,N-dimethylformamide, acetonitrile and water as well as mixed solvents thereof.

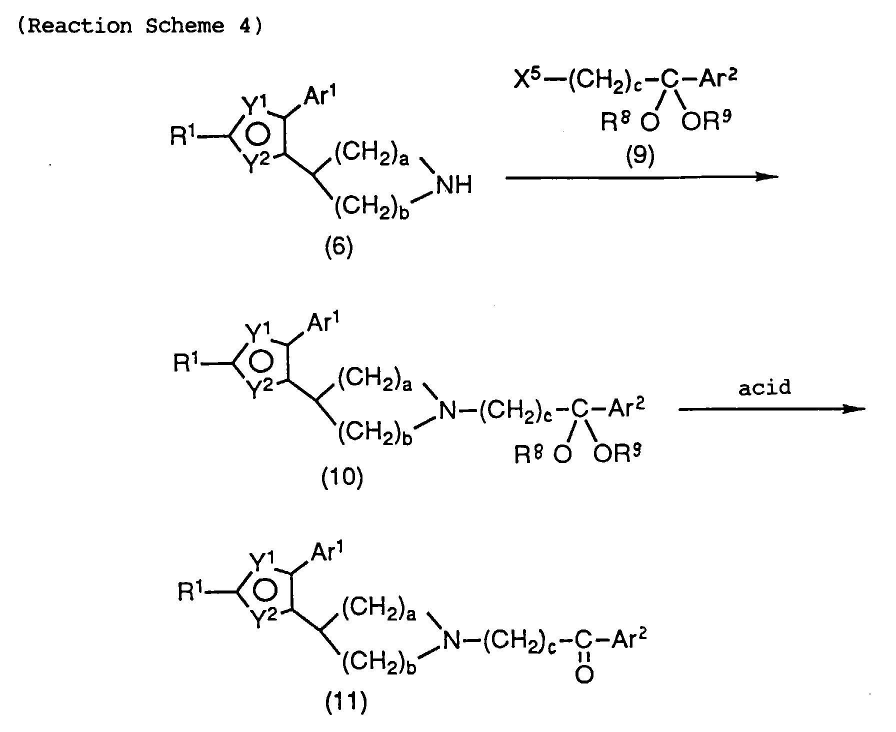

The halide containing a ketal represented by formula (9) and NH derivative (6) are reacted in the same manner as described above whereby compound (10) of the present invention can be obtained.

Then, compound (10) can be reacted with an acid in an inert solvent to obtain compound (11) of the present invention.

The acid mentioned here includes e.g. inorganic acids such as hydrochloric acid, sulfuric acid etc., and organic acids such as p-toluenesulfonic acid, trifluoroacetic acid etc.

The inert solvent includes e.g. organic acids such as acetic acid etc., organic halogen compounds such as chloroform etc., alcohols such as methanol, ethanol, isopropanol etc., ethers such as diethyl ether, tetrahydrofuran, dioxane etc., hydrocarbons such as toluene etc., ketone compounds such as acetone, methyl ethyl ketone etc., N,N-dimethylformamide, acetonitrile, and water as well as mixed solvents thereof.

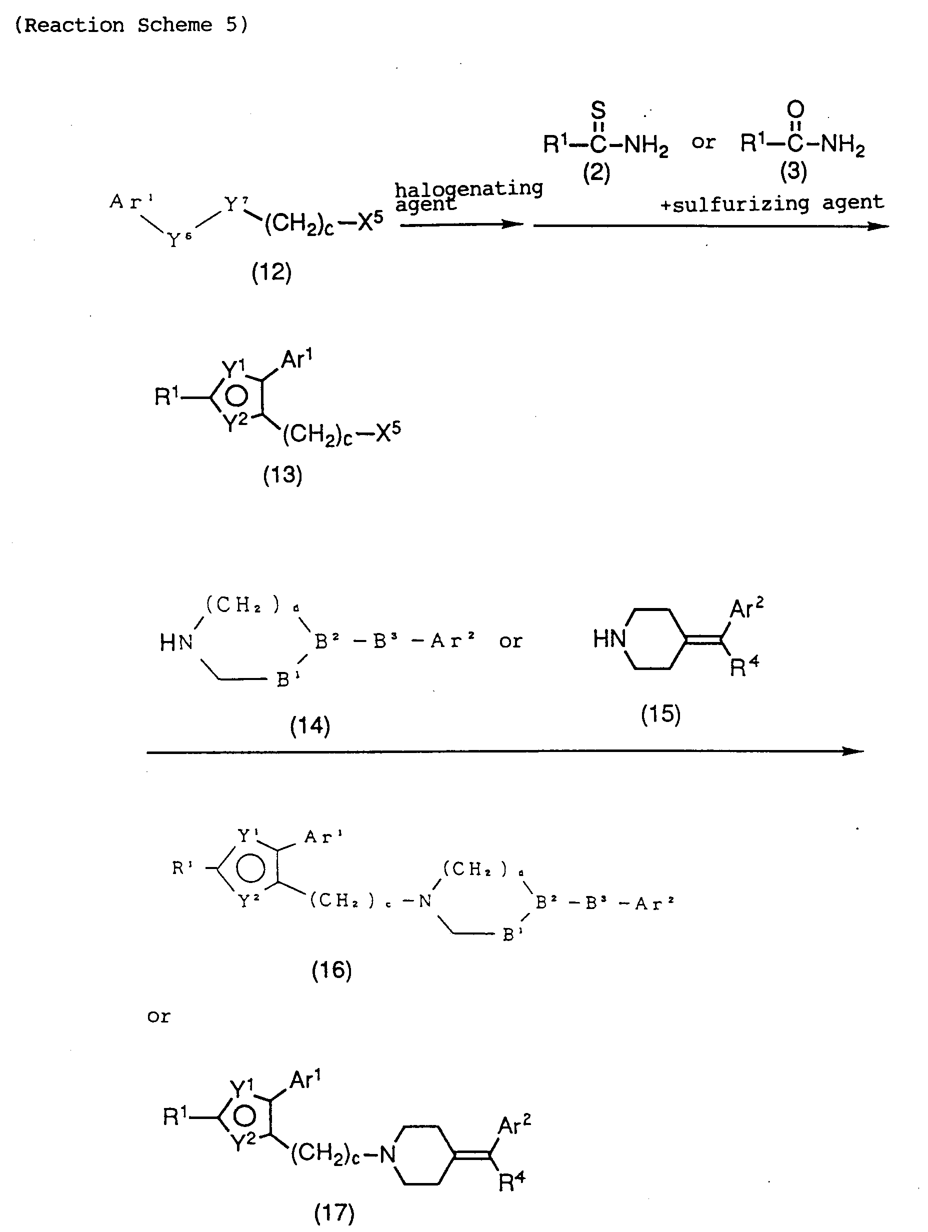

Ketone derivative (12) is halogenated with a halogenating agent in the same manner as in reaction scheme 1 and then reacted with a thiourea derivative or thioamide derivative represented by formula (2), or with an urea derivative or amide derivative represented by formula (3) along with a sulfurizing agent to give halide derivative (13) which is then reacted with amine derivative (14) or (15) in the presence of a base in an inert solvent whereby compound (16) or (17) of the present invention can be obtained.

The base mentioned here includes organic amines such as triethylamine, diisopropylethylamine, pyridine etc., alcoholates such as sodium ethoxide etc., alkali metal amides such as sodium amide etc., inorganic bases such as sodium hydrogen carbonate, sodium carbonate, potassium carbonate, sodium hydroxide, sodium hydride, and organic salts such as sodium acetate etc.

The inert solvent includes e.g. organic carboxylic acids such as acetic acid, organic halogen compounds such as chloroform, carbon tetrachloride etc. alcohols such as methanol, ethanol, isopropanol etc., ethers such as tetrahydrofuran, dioxane etc., hydrocarbons such as benzene, toluene etc., ketone compounds such as acetone, methyl ethyl ketone etc., N,N-dimethylformamide, acetonitrile, and water as well as mixed solvents thereof.

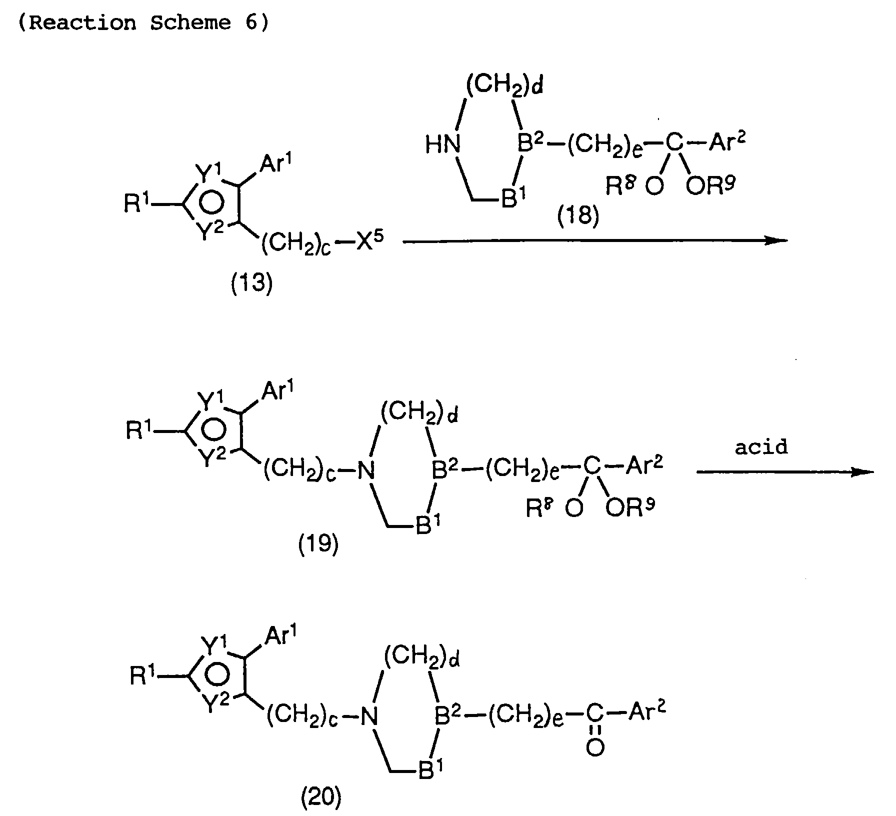

In addition, the amine derivative containing a ketal represented by formula (18) and halide derivative (13) are reacted in the same manner as described above whereby compound (19) of the present invention can be obtained.

Then, compound (19) can be reacted with an acid in an inert solvent to obtain compound (20) of the present invention.

The acid mentioned here includes e.g. inorganic acids such as hydrochloric acid, sulfuric acid etc. and organic acids such as p-toluenesulfonic acid, trifluoroacetic acid etc.

The inert solvent includes e.g. organic acids such as acetic acid, organic halogen compounds such as chloroform etc., alcohols such as methanol, ethanol, isopropanol etc., ethers such as diethyl ether, tetrahydrofuran, dioxane etc., hydrocarbons such as toluene etc., ketone compounds such as acetone, methyl ethyl ketone etc., N,N-dimethylformamide, acetonitrile, and water as well as mixed solvents thereof.

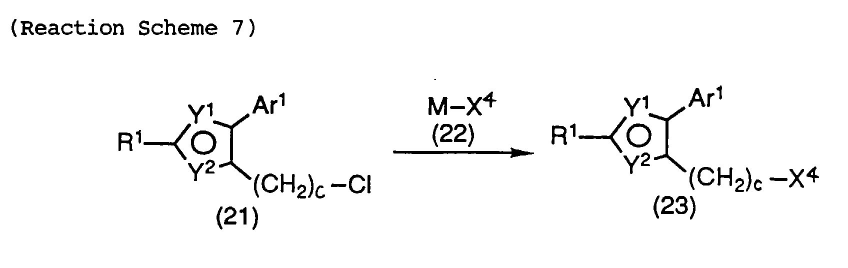

A compound represented by formula (21) which is equivalent to the compound of formula (13) wherein X⁵ is a chlorine atom is reacted with an inorganic halide represented by formula (22) in an inert solvent whereby the compound of formula (23) wherein the chlorine atom has been replaced by another halogen atom can be obtained.

The inert solvent mentioned here includes e.g. organic acids such as acetic acid, organic halogen compounds such as chloroform etc., alcohols such as ethanol, isopropanol etc., ethers such as diethyl ether, tetrahydrofuran, dioxane etc., hydrocarbons such as toluene etc., ketones such as acetone, methyl ethyl ketone etc., N,N-dimethylformamide, acetonitrile, and water as well as mixed solvents thereof.

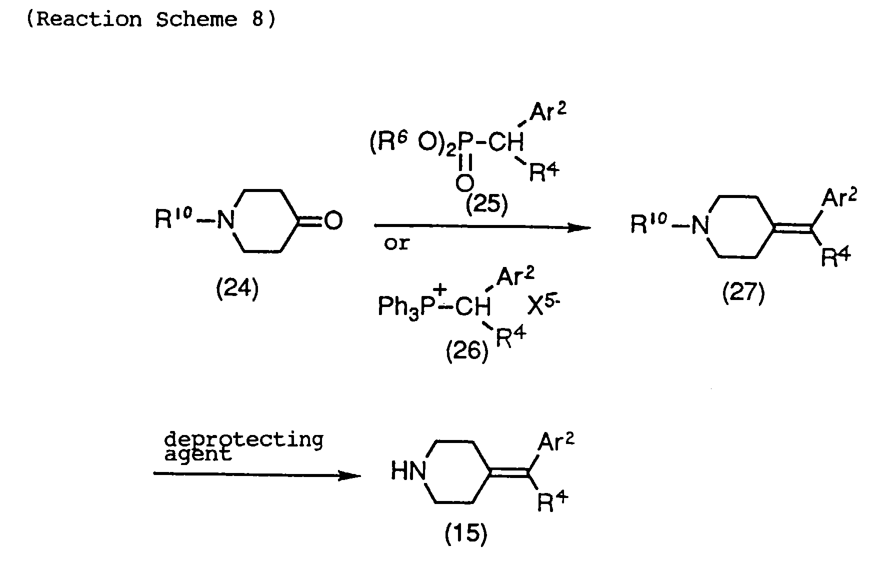

4-Benzylidenepiperidine derivative of formula (15) is obtained by condensing piperidone derivative (24) with dialkyl arylmethylphosphonate (25) or triphenyl(arylmethyl)phosphonium salt (26) in the presence of a base in an inert solvent to convert it into compound (27) and then removing the protecting group of compound (27) with a deprotecting agent.

The base mentioned here includes sodium hydride, potassium hydride, sodium methoxide, potassium t-butoxide, n-butyl lithium, lithium diisopropylamide, lithium bis(trimethylsilyl)amide, sodium amide etc., and if necessary a catalyst such as 15-crown-5 ether, 18-crown-6 ether etc. is used in combination.

The inert solvent includes e.g. ethers such as diethyl ether, tetrahydrofuran, dioxane etc., hydrocarbons such as benzene, toluene etc., alcohols such as ethanol etc.

The reaction solvent used for deprotection includes ethers such as diethyl ether, tetrahydrofuran, dioxane etc., hydrocarbons such as benzene, toluene etc., alcohols such as ethanol etc., organic carboxylates such as ethyl acetate, ketones such as acetone etc., alkyl halides such as dichloromethane, chloroform etc., organic carboxylic acids such as acetic acid etc., N,N-dimethylformamide and water as well as mixed solvent thereof.

The deprotecting agent except for the case where R¹⁰ is not a C₁ to C₅ alkyl group includes e.g. inorganic acids such as hydrochloric acid, hydrobromic acid, sulfuric acid etc., organic acids such as trifluoroacetic acid, formic acid, methanesulfonic acid etc., and acids such as a solution of hydrogen chloride in dioxane or in ethyl acetate, a solution of hydrogen bromide in acetic acid and bases e.g. inorganic bases such as sodium hydroxide, potassium hydroxide, barium hydroxide etc. In case R¹⁰ is a C₁ to C₅ alkyl group, compound (27) is reacted with a C₂ to C₆ alkoxycarbonyl halide in the presence or absence of bases such as triethylamine, di-isopropylamine, potassium carbonate etc. and then the resulting reaction product is deprotected with said deprotecting agent.

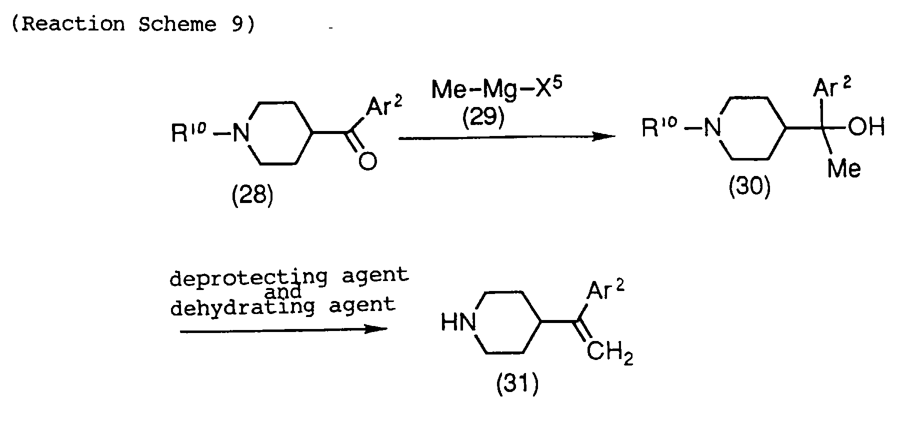

Ketone derivative (28) is reacted with Grignard reagent (29) to convert into tertiary alcohol (30) which is then deprotected and dehydrated simultaneously or in different steps whereby intermediate (31) for synthesis of the compounds of the present invention can be obtained.

The deprotecting agent in case R¹⁰ is not a C₁ to C₅ alkyl group includes e.g. inorganic acids such as hydrochloric acid, hydrobromic acid, sulfuric acid etc., organic acids such as trifluoroacetic acid, formic acid, methanesulfonic acid etc., and acids such as a solution of hydrogen chloride in dioxane or in ethyl acetate, a solution of hydrogen bromide in acetic acid, and bases e.g. inorganic bases such as sodium hydroxide, potassium hydroxide, barium hydroxide etc. In case R¹⁰ is a C₁ to C₅ alkyl group, compound (30) is reacted with a C₂ to C₆ alkoxycarbonyl halide in the presence or absence of bases such as triethylamine, diisopropylamine, potassium carbonate etc. and then deprotected with said deprotecting agent.

The dehydrating agent includes e.g. inorganic acids hydrochloric acid, hydrobromic acid, sulfuric acid etc., organic acids such as trifluoroacetic acid, formic acid, methanesulfonic acid etc., and acids such as a solution of hydrogen chloride in dioxane or in ethyl acetate and a solution of hydrogen bromide in acetic acid. The alcohol of formula (30) may be subjected to acylation such as acetylation or sulfonation such as mesylation and then dehydrated in organic amines such as triethylamine, di-isopropylethylamine, pyridine etc., alcoholates such as sodium ethoxide etc., alkali metal amides such as sodium amide etc., inorganic bases such as sodium hydrogen carbonate, sodium carbonate, potassium carbonate, sodium hydroxide, sodium hydride etc., and bases of organic salts such as sodium acetate etc.

The solvent for the Grignard reagent includes ethers such as diethyl ether, tetrahydrofuran, dioxane etc., and hydrocarbons such as benzene, toluene etc.

The reaction solvent used for deprotection includes ethers such as diethyl ether, tetrahydrofuran, dioxane etc., hydrocarbons such as benzene, toluene etc., alcohols such as ethanol etc., organic carboxylates such as ethyl acetate etc., ketones such as acetone etc., alkyl halides such as dichloromethane, chloroform etc., organic carboxylic acids such as acetic acid etc., N,N-dimethylformamide and water as well as mixed solvents thereof.

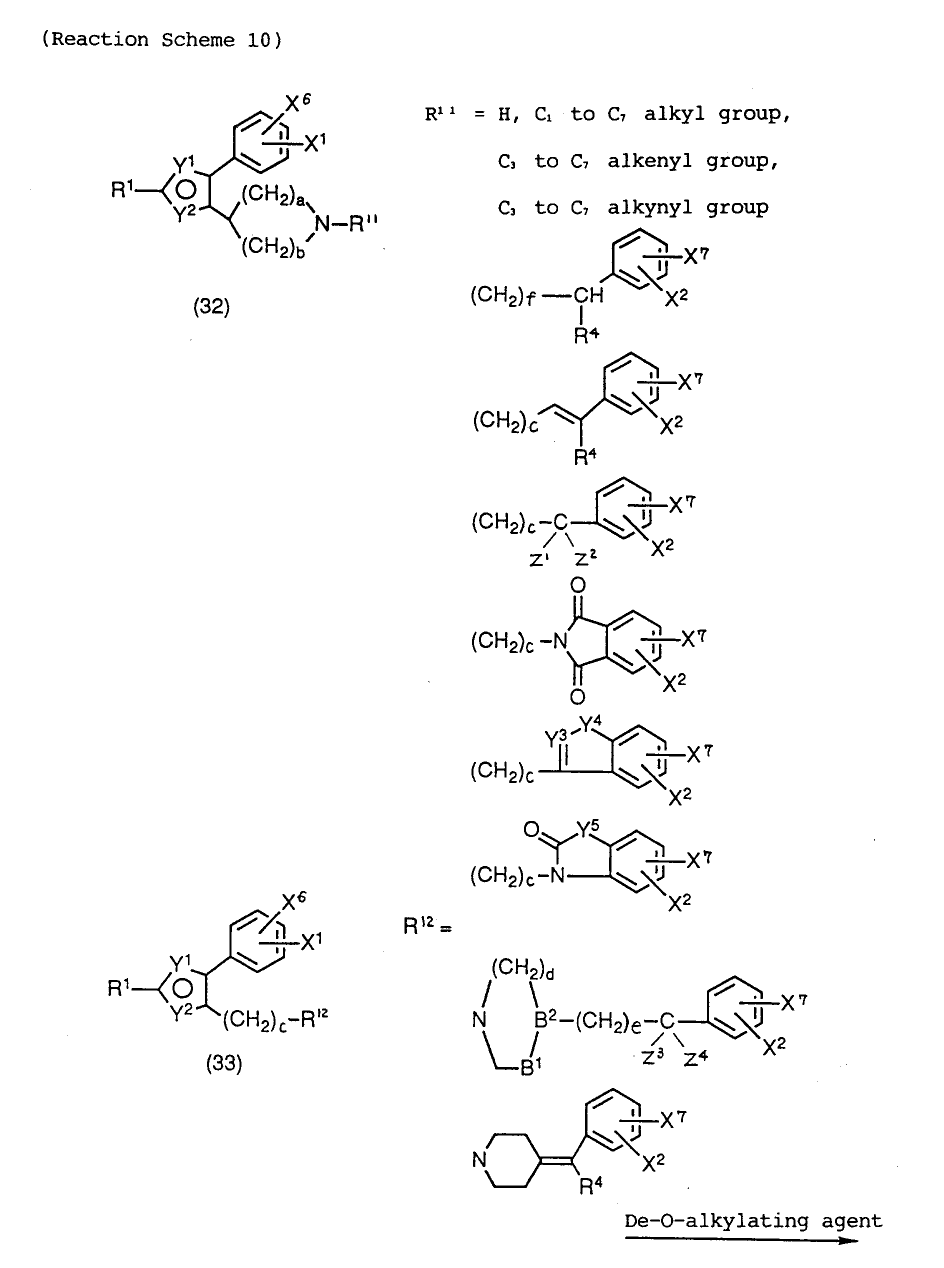

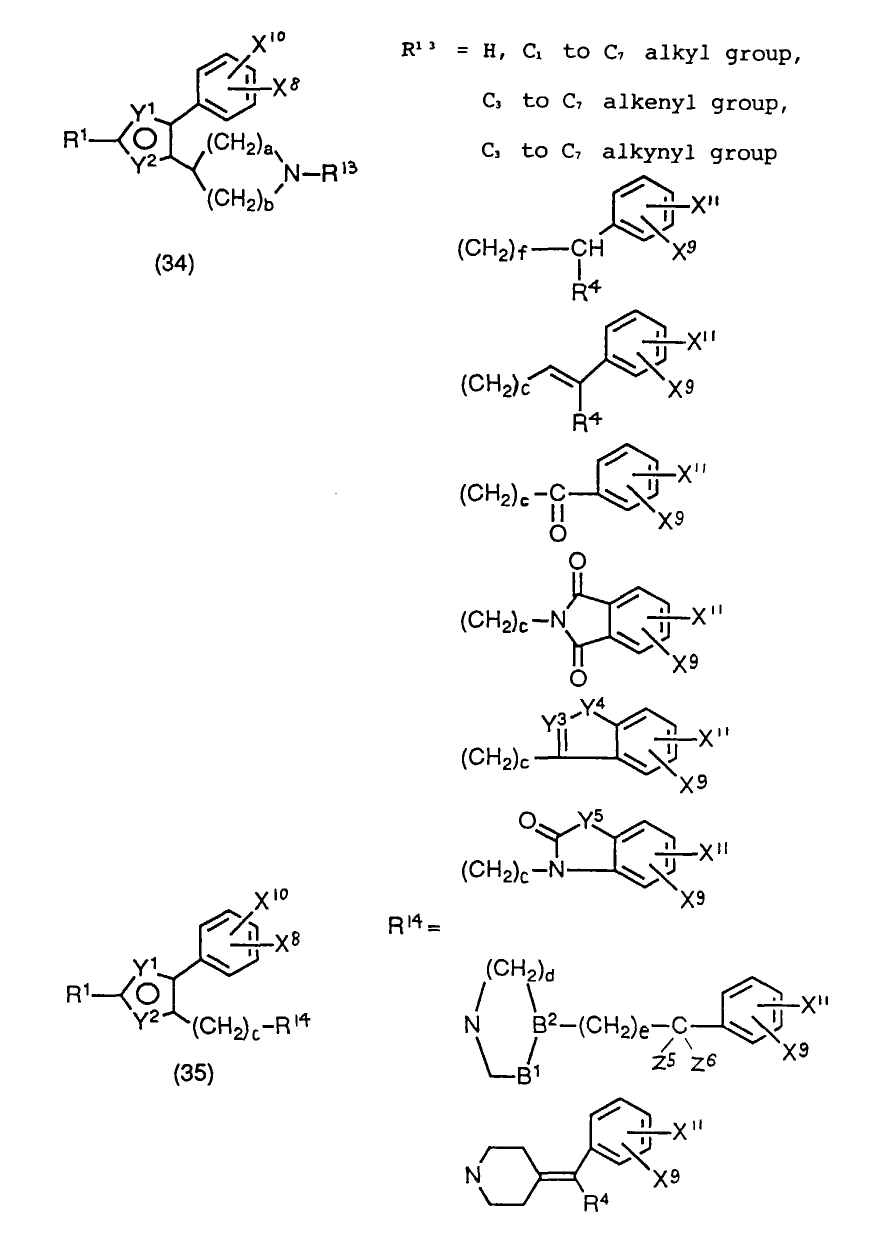

Phenol derivative (34) or (35) as the compound of the present invention is obtained by allowing to react the corresponding alkoxy derivative (32) or (33) with the de-O-alkylating agent such as hydrobromic acid or boron tribromide in an inert solvent.

The inert solvent mentioned here includes hydrocarbons such as benzene, toluene etc., alkyl halides such as dichloromethane, chloroform etc., alcohols such as ethanol etc., organic carboxylic acids such as acetic acid etc., and water as well as mixed solvents thereof.

The compounds of the present invention demonstrate an excellent affinity for dopamine D₄ receptor while having a low affinity for dopamine D₂ receptor, thus showing excellent separability.

Therefore, the compounds of the present invention are useful for preventing and treating diseases such as problematic behaviors etc. accompanying schizophrenia, cerebrovascular disease and senile dementia, and useful as agents not causing extrapyramidal disorder as side effects.

For the above-described object, the compounds of the present invention can be formed into tablets, pills, capsules, granules, powder, liquid preparation, emulsion, suspension, injection etc. in usual pharmaceutical techniques by adding conventional fillers, binders, disintegrator, pH adjustor, solubilizer etc.

The compounds of the present invention can be administered orally or parenterally to an adult patient at a dose of 0.1 to 500 mg/day at once or in divided portions. This dose can be arbitrarily altered depending on a type of disease, and the age, weight and disease severeness of the patient.

### Best Mode for Carrying Out the Invention

Hereinafter, the present invention is described in more detail by reference to the Examples and Test Examples.

### Example 1

### Synthesis of 2-amino-5-(1-benzylpyrrolidin-3-yl)-4-(4-fluorophenyl)thiazole

2.01 g of 1-benzyl-3-[2-(4-fluorophenyl)-2-oxoethyl]pyrrolidine maleate was distributed between 2 N aqueous sodium hydroxide and diethyl ether, and the diethyl ether layer was washed with saturated aqueous sodium chloride and then dried over anhydrous sodium sulfate. The desiccant was removed by filtration, and 1.5 ml of 4 N hydrogen chloride in ethyl acetate was added and the solvent was distilled away under reduced pressure.

The residue was dissolved in 10 ml acetic acid and 5 ml of acetic acid solution containing 781 mg of bromine was added dropwise for 30 minutes. This reaction mixture was stirred for 2 hours at room temperature and the acetic acid was removed under reduced pressure. To this residue were added 10 ml of ethanol and 405 mg of thiourea, and the mixture was refluxed under heating for 18 hours. The reaction mixture was concentrated under reduced pressure and distributed between 0.5 N aqueous sodium hydroxide and dichloromethane. The aqueous layer was further extracted twice with dichloromethane, and the extracts were combined and dried over anhydrous sodium sulfate. The desiccant was removed by filtration and then the filtrate was concentrated under reduced pressure and purified by flash column chromatography (silica gel: Chromatorex NHDM1020 (Fuji-Davison Chemical Ltd.), eluent: hexane/ethyl acetate = 10 : 1 - 2 : 1) and recrystallized from dichloromethane to give 1.33 g of 2-amino-5-(1-benzylpyrrolidin-3-yl)-4-(4-fluorophenyl) thiazole.
m.p. 125.5 - 126.5°C

The structures and physical data of this compound and compounds obtained in the same manner are shown in Table B.

### Example 2

### Synthesis of 2-amino-4-(4-fluorophenyl)-5-(pyrrolidin-3-yl)thiazole

6.90 g of 3-[2-(4-fluorophenyl)-2-oxoethyl]pyrrolidine hydrochloride was dissolved in 35 ml of acetic acid, and 5 ml of acetic acid solution containing 4.30 g of bromine was added dropwise for 30 minutes. This reaction mixture was stirred for 3 hours at room temperature and the acetic acid was removed under reduced pressure. To this residue were added 35 ml of ethanol and 2.24 g of thiourea, and the mixture was refluxed under heating for 15 hours. The reaction mixture was concentrated under reduced pressure and distributed between 2 N aqueous sodium hydroxide and tetrahydrofuran. The aqueous layer was further extracted twice with tetrahydrofuran, and the extracts were combined and dried over anhydrous sodium sulfate. The desiccant was removed by filtration and then the filtrate was concentrated under reduced pressure and purified by flash column chromatography (silica gel : Chromatorex NHDM1020 (Fuji-Davison Chemical Ltd.), eluent: chloroform/ethanol = 50 : 1 - 20 : 1) and recrystallized from dichloromethane to give 4.80 g of 2-amino-4-(4-fluorophenyl)-5-(pyrrolidin-3-yl)thiazole.
m.p. 176.5 - 178.0 °C

The structures and physical data of this compound and compounds obtained in the same manner are shown in Table A.

### Example 3

### Synthesis of (+)-2-amino-4-(4-fluorophenyl)-5-(pyrrolidin-3-yl)thiazole dihydrobromide

(1) 156.3 g of 3-[2-(4-fluorophenyl)-2-oxoethyl]pyrrolidine hydrochloride was distributed under cooling on ice between 0.5 N aqueous sodium hydroxide and chloroform. The aqueous layer was further extracted twice with chloroform, and the extracts thus obtained were combined, washed with water and then with saturated aqueous sodium chloride, and dried over anhydrous potassium carbonate. The desiccant was removed by filtration and then the filtrate was concentrated under reduced pressure. The resulting residue was dissolved in ethanol and added to an ethanol solution of 277.5 g of (+)-di-p-toluoyltartaric acid under stirring. The precipitated crystal was collected by filtration and recrystallized 3 times from ethanol to give 75.5 g of a crystal. This crystal was distributed between dichloromethane and 0.5 N aqueous sodium hydroxide, and the organic layer was further washed with 0.5 N aqueous sodium hydroxide and then with saturated aqueous sodium chloride. The organic layer was dried over anhydrous sodium sulfate and the desiccant was removed by filtration. 18.6 g of diisopropylethylamine was added to the filtrate and 14.6 g of ethyl chlorocarbonate was added dropwise for 30 minutes under cooling on ice. This reaction mixture was washed twice with 1 N hydrochloric acid and then washed with saturated aqueous sodium chloride, and the organic layer was dried over anhydrous magnesium sulfate. The desiccant was removed by filtration, and the filtrate was concentrated under reduced pressure and purified by flash column chromatography (silica gel : Wako Gel C200, eluent: hexane/ethyl acetate = 3 : 1) to give 31.3 g of (+)-1-ethoxycarbonyl-3-[2-(4-fluorophenyl)-2-oxoethyl]pyrrolidine.
   NMR (CDCl₃) δ (ppm); 1.25 (3H, t, J = 7.1 Hz), 1.50 - 1.69 (1H, m), 2.11 - 2.19 (1H, m), 2.70 - 2.84 (1H, m), 2.95 - 3.14 (3H, m), 3.29 - 3.61 (2H, m), 3.73 (1H, dd, J = 10.7 Hz, 7.1 Hz), 4.13 (2H, q, J = 7.1 Hz), 7.08 - 7.20 (2H, m), 7.93 - 8.03 (2H, m)
   MS m/e: 280 (M⁺+1, 100 %)
   [α]_{D}²⁹ = +9.07 (c 0.881, MeOH)

   The first filtrate was concentrated under reduced pressure and distributed between 0.5 N aqueous sodium hydroxide and chloroform. Further, the organic layer was washed with 0.5 N aqueous sodium hydroxide and then with saturated aqueous sodium chloride, and dried over anhydrous potassium carbonate. The desiccant was removed by filtration, and the filtrate was concentrated under reduced pressure. The resulting residue was dissolved in ethanol and added to an ethanol solution of 111.0 g of (-)-di-p-toluoyltartaric acid under stirring. The same procedure as described above was repeated in the subsequent step whereby 27.6 g of (-)-1-ethoxycarbonyl-3-[2-(4-fluorophenyl)-2-oxoethyl]pyrrolidine was obtained.
   NMR (CDCl₃) δ (ppm): 1.25 (3H, t, J = 7.1 Hz), 1.50 - 1.69 (1H, m), 2.11 - 2.19 (1H, m), 2.70 - 2.84 (1H, m), 2.95 - 3.14 (3H, m), 3.29 - 3.61 (2H, m), 3.73 (1H, dd, J = 10.7 Hz, 7.1 Hz), 4.13 (2H, q, J = 7.1 Hz), 7.08 - 7.20 (2H, m), 7.93 - 8.03 (2H, m)
   MS m/e: 280 (M⁺+1, 100 %)
   [α]_{D}²⁹ = -8.65 (c 0.748, MeOH)
(2) 17.0 g of (+)-1-ethoxycarbonyl-3-[2-(4-fluorophenyl)-2-oxoethyl]pyrrolidine was dissolved in 85 ml of acetic acid, and 0.85 ml of 47 % aqueous hydrobromic acid was added. Then, 9.73 g of bromine was added dropwise over a period of 30 minutes at room temperature. This reaction mixture was stirred at room temperature for 2 hours, and the acetic acid was distilled away under reduced pressure.
   To this residue were added 85 ml of ethanol and 5.56 g of thiourea, and the mixture was refluxed under heating for 2 hours. The reaction mixture was concentrated under reduced pressure and distributed between 0.5 N aqueous sodium hydroxide and ethyl acetate. The organic layer was washed with saturated aqueous sodium chloride and dried over anhydrous sodium sulfate. The desiccant was removed by filtration and then the filtrate was concentrated under reduced pressure and purified by flash column chromatography (silica gel : Wako Gel C200, eluent: hexane/ethyl acetate = 3 : 1 - 1 : 1) and recrystallized from diisopropyl ether to give 12.0 g of (+)-2-amino-4-(4-fluorophenyl)-5-(1-ethoxycarbonylpyrrolidin-3-yl)thiazole.
   m.p. 184.0 - 185.0 °C
   [α]_{D}²⁸ = +47.6 (c 0.903, CHCl₃)
(3) 47 % aqueous hydrobromic acid was added to 11.8 g of (+)-2-amino-4-(4-fluorophenyl)-5-(1-ethoxycarbonylpyrrolidin-3-yl)thiazole, and the mixture was refluxed for 1.5 hours under heating. The reaction mixture was concentrated under reduced pressure and recrystallized from isopropanol to give 14.7 g of (+)-2-amino-4-(4-fluorophenyl)-5-(pyrrolidin-3-yl)thiazole dihydrobromide.
   m.p. 257.0 - 259.0°C (decomposed)
   [α]_{D}²⁹ = +14.8 (c 1.03, MeOH)

   The structures and physical data of this compound and compounds obtained in the same manner are shown in Tables A and F.

### Example 4

### Synthesis of 2-amino-4-(4-fluorophenyl)-5-[1-[4-(4-fluorophenyl)-4-oxobutyl]pyrrolidin-3-yl]thiazole dihydrochloride

400 mg of 2-amino-4-(4-fluorophenyl)-5-(pyrrolidin-3-yl)thiazole, 500 mg of 4-chloro-4'-fluorobutyrophenone and 1.20 ml of triethylamine were stirred in 2 ml of N,N-dimethylformamide at 50 °C for 5 days. This reaction mixture was poured into water, then extracted with ethyl acetate and washed with water and then with saturated aqueous sodium chloride. The extract was dried over anhydrous sodium sulfate, and the desiccant was removed by filtration, and the filtrate was concentrated under reduced pressure and purified by flash column chromatography (silica gel : Wako Gel C200, eluent: chloroform/ethanol = 50 : 1 - 20 : 1) and recrystallized from ethyl acetate to give 261 mg of 2-amino-4-(4-fluorophenyl)-5-[1-[4-(4-fluorophenyl)-4-oxobutyl]pyrrolidin-3-yl]thiazole.
m.p. 145.0 - 146.0°C

200 mg of 2-amino-4-(4-fluorophenyl)-5-[1-[4-(4-fluorophenyl)-4-oxobutyl]pyrrolidin-3-yl]thiazole was dissolved in 10 ml of chloroform, and 0.3 ml of 4 N hydrogen chloride solution in dioxane was added, and the solvent was distilled away under reduced pressure and the residue was recrystallized from isopropanol to give 194 mg of 2-amino-4-(4-fluorophenyl)-5-[1-[4-(4-fluorophenyl)-4-oxobutyl]pyrrolidin-3-yl]thiazole dihydrochloride.
m.p. 146.0 - 147.5°C

The structures and physical data of this compound and compounds obtained in the same manner are shown in Tables A, B, C, D, E, G and H.

### Example 5

### Synthesis of (+)-2-amino-4-(4-fluorophenyl)-5-[1-[4-(4-fluorophenyl)-4-oxobutyl]pyrrolidin-3-yl]thiazole

(1) 4.50 g of (+)-2-amino-4-(4-fluorophenyl)-5-(pyrrolidin-3-yl)thiazole dihydrobromide, 2.74 g of 2-(3-chloropropyl)-2-(4-fluorophenyl)-1,3-dioxolane and 4.60 g of diisopropylethylamine were added to 4.5 ml of methanol and the mixture was refluxed under heating for 14 hours. The reaction mixture was concentrated under reduced pressure and distributed between 0.5 N aqueous sodium hydroxide and chloroform. The organic layer was further washed with saturated aqueous sodium chloride and dried over anhydrous sodium sulfate. The desiccant was removed by filtration, and the filtrate was concentrated under reduced pressure and purified by flash column chromatography (silica gel : Chromatorex NHDM1020 (Fuji-Davison Chemical Ltd.), eluent: hexane/ethyl acetate = 3 : 1 - 1 : 1) to give 4.07 g of an oily product.
(2) 25 ml of 1 N hydrochloric acid and 25 ml of tetrahydrofuran were added to the above oily product and the mixture was stirred at room temperature for 15 hours. The reaction mixture was concentrated under reduced pressure and distributed between 2 N aqueous sodium hydroxide and chloroform. The organic layer was further washed with saturated aqueous sodium chloride and dried over anhydrous sodium sulfate. The desiccant was removed by filtration, and the filtrate was concentrated under reduced pressure and purified by flash column chromatography (silica gel : Chromatorex NHDM1020 (Fuji-Davison Chemical Ltd.), eluent: hexane/ethyl acetate = 3 : 1 - 1 : 1) and recrystallized from ethyl acetate to give 2.64 g of (+)-2-amino-4-(4-fluorophenyl)-5-[1-[4-(4-fluorophenyl)-4-oxobutyl]pyrrolidin-3-yl]thiazole.
   m.p. 112.0 - 113.0°C
   [α]_{D}²⁸ = +17.6 (c 0.987, CHCl₃)

   The structures and physical data of this compound and compounds obtained in the same manner are shown in Tables D, E, F and H.

### Example 6

### Synthesis of 2-amino-4-(4-fluorophenyl)-5-[1-[3-(6-fluoro-1,2-benzisoxazol-3-yl)propyl]pyrrolidin-3-yl]thiazole dihydrochloride

1.00 g of 2-amino-4-(4-fluorophenyl)-5-(pyrrolidin-3-yl)thiazole, 993 mg of 3-(3-chloropropyl)-6-fluoro-1,2-benzisoxazole and 1.26 g of diisopropylethylamine were stirred in 5 ml of N,N-dimethylformamide at 50°C for 5 days. This reaction mixture was poured into water, extracted with ethyl acetate, and washed with water. The organic layer was extracted with 1 N hydrochloric acid and the resulting aqueous layer was made basic with 10 % aqueous sodium hydroxide and extracted again with ethyl acetate. The extract was washed with water and then with saturated aqueous sodium chloride and dried over anhydrous sodium sulfate. The desiccant was removed by filtration, and the filtrate was concentrated under reduced pressure, and the residue was recrystallized from ethyl acetate to give 530 mg of 2-amino-4-(4-fluorophenyl)-5-[1-[3-(6-fluoro-1,2-benzisoxazol-3-yl)propyl]pyrrolidin-3-yl]thiazole.
m.p. 125.5 - 127.0°C

400 mg of 2-amino-4-(4-fluorophenyl)-5-[1-[3-(6-fluoro-1,2-benzisoxazol-3-yl)propyl]pyrrolidin-3-yl]thiazole was dissolved in 10 ml of chloroform, and 1.0 ml of 4 N hydrogen chloride in dioxane was added, and the solvent was distilled away under reduced pressure, and the residue was recrystallized from isopropanol to give 441 mg of 2-amino-4-(4-fluorophenyl)-5-[1-[3-(6-fluoro-1,2-benzisoxazol-3-yl)propyl]pyrrolidin-3-yl]thiazole dihydrochloride.
m.p. 148.5 - 150.0°C

The structures and physical data of this compound and compounds obtained in the same manner are shown in Tables E and F.

### Example 7

### Synthesis of 2-amino-4-(4-fluorophenyl)-5-[1-[3-(6-fluoro-1,2-benzisothiazol-3-yl)propyl]pyrrolidin-3-yl]thiazole

(1) 12.5 g of 2-(4-fluorophenyl)-4,4-dimethyloxazoline was dissolved in 200 ml of tetrahydrofuran, and 40 ml of 1.69 M n-butyl lithium in hexane under cooling at -40 °C was added dropwise. The mixture was further stirred at a temperature of -40 °C for 90 minutes, and 17.5 ml of dimethyl disulfide was added dropwise and the mixture was further stirred for 2 hours at a temperature of -40 to -30°C. The reaction mixture was poured into water and then extracted twice with diethyl ether. The organic layers were combined and washed with saturated aqueous sodium chloride and dried over anhydrous magnesium sulfate. The desiccant was removed by filtration, and the filtrate was concentrated under reduced pressure. The residue was purified by flash column chromatography (silica gel : Merk Kiesel Gel 60, 230 - 400 mesh, eluent: hexane/ethyl acetate = 17 : 3) to give 11.3 g of 2-(4-fluoro-2-methylthiophenyl)-4,4-dimethyloxazoline.
   m.p. 71.0 °C
(2) A mixture of 10.0 g of 2-(4-fluoro-2-methylthiophenyl)-4,4-dimethyloxazoline, 20 ml of methyl iodide and 40 ml of acetone was refluxed under heating for 16 hours. After cooling, the reaction mixture was concentrated under reduced pressure, and diethyl ether was added and the resulting crystal was collected by filtration whereby 15.8 g of 2-(4-fluoro-2-methylthiophenyl)-4,4-dimethyl-N-methyl-2-oxazolinium iodide was obtained.
   m.p. 190.0 - 191.0°C
(3) 14.5 g of 2-(4-fluoro-2-methylthiophenyl)-4,4-dimethyl-N-methyl-2-oxazolinium iodide was suspended in 150 ml of ethanol, and 1.43 g of sodium borohydride under cooling at -10 to -5°C was added with small portions. The mixture was stirred at the same temperature for 30 minutes, then 100 ml of 2 N hydrochloric acid was added, and the mixture was stirred at room temperature for additional 1 hour. The reaction mixture was concentrated under reduced pressure, and the residue was distributed between water and chloroform. The aqueous layer was further extracted with chloroform, and the organic layers were combined, washed with water and dried over anhydrous magnesium sulfate. The desiccant was removed by filtration, and the filtrate was concentrated under reduced pressure and purified by flash column chromatography (silica gel : Merk Kiesel Gel 60, 230 - 400 mesh, eluent: hexane/ethyl acetate = 17 : 3) to give 4.55 g of 4-fluoro-2-methylthiobenzaldehyde.

   m.p. 62.0 - 63.0°C
(4) 0.62 g of lithium was suspended in 50 ml of ether, and 1.0 g of 3-[(1-ethoxy)ethoxy]propyl bromide was added dropwise at room temperature. When the reaction solution turned turbid, the reaction solution was cooled to -10 °C, and 7.36 g of 3-[(1-ethoxy)ethoxy]propyl bromide was added dropwise over a period of 45 minutes. After dropwise addition, the mixture was further stirred for 1 hour at a temperature of -20 to -15°C. Then the reaction solution was cooled to -60 °C, and 30 ml of diethyl ether solution containing 4.50 g of 4-fluoro-2-methylthiobenzaldehyde was added dropwise. The reaction temperature was gradually raised to -20°C, and 2 ml of methanol was added. The reaction mixture was poured into saturated aqueous ammonium chloride and extracted twice with diethyl ether. The organic layers were combined, washed with saturated aqueous sodium chloride and dried over anhydrous magnesium sulfate. The desiccant was removed by filtration, and the filtrate was concentrated under reduced pressure and purified by flash column chromatography (silica gel : Merk Kiesel Gel 60, 230 - 400 mesh, eluent: hexane/ethyl acetate = 4 : 1) to give 7.14 g of 1-(4-fluoro-2-methylthiophenyl)-4-[(1-ethoxy)ethoxy]butanol.
   NMR (CDCl₃) δ (ppm): 1.21 (3H, t, J = 7.2 Hz), 1.33 (3H, d, J = 5.4 Hz), 1.68 - 1.82 (3H, m), 1.82 - 1.93 (1H, m), 2.47 (3H, s), 2.89 - 2.94 (1H, br), 3.43 - 3.52 (2H, m), 3.60 - 3.69 (2H, m), 4.72 (1H, q, J = 5.4 Hz), 5.03 - 5.10 (1H, m), 6.82 - 6.91 (2H, m), 7.43 - 7.50 (1H, m)
(5) 4.4 ml of oxalyl chloride was dissolved in 70 ml of dichloromethane and while the reaction temperature was maintained at -60 to -50 °C, 10 ml of dichloromethane solution containing 7.9 ml of dimethyl sulfoxide was added dropwise. After dropwise addition, the mixture was stirred at -60°C for 3 minutes, and 10 ml dichloromethane solution containing 7.0 g of 1-(4-fluoro-2-methylthiophenyl)-4-[(1-ethoxy)ethoxy]butanol was added dropwise. After dropwise addition, the mixture was further stirred -60°C for 15 minutes, and then 16 ml of triethylamine was added dropwise, and the reaction temperature was gradually raised to 10°C. Ice water was added to the reaction solution to separate the organic layer, and the aqueous layer was further extracted with chloroform. The organic layers were combined, washed with water and dried over anhydrous magnesium sulfate. The desiccant was removed by filtration, and the filtrate was concentrated under reduced pressure and purified by flash column chromatography (silica gel: Merk Kiesel Gel 60, 230 - 400 mesh, eluent: hexane/ethyl acetate = 4 : 1) to give 5.18 g of 4-[(1-ethoxy)ethoxy]-4'-fluoro-2'-methylthiobutyrophenone.
   NMR (CDCl₃) δ (ppm): 1.20 (3H, t, J = 7.0 Hz), 1.30 (3H, d, J = 5.3 Hz), 1.99 - 2.08 (2H, m), 2.43(3H, s), 3.06 (3H, t, J = 7.1 Hz), 3.43 - 3.56 (2H, m), 3.57 - 3.70 (2H, m), 4.68 (1H, q, J = 5.3 Hz), 6.83 - 6.90 (1H, m), 6.97 - 7.04 (1H, m), 7.89 - 7.98 (1H, m)
(6) A mixture of 5.10 g of 4-[(1-ethoxy)ethoxy]-4'-fluoro-2'-methylthiobutyrophenone, 1.61 g of hydroxylamine hydrochloride and 10 ml of pyridine was stirred at room temperature for 2 days. The reaction solution was distributed between water and ethyl acetate, and the aqueous layer was further extracted with ethyl acetate. The organic layers were combined, washed with saturated aqueous sodium chloride and dried over anhydrous magnesium sulfate. The desiccant was removed by filtration, and the filtrate was concentrated under reduced pressure. The residue was dissolved in 40 ml of tetrahydrofuran, and 4 ml of 1 N hydrochloric acid was added and the mixture was stirred at room temperature for 4 hours. The solvent was distilled away under reduced pressure, and the resulting residue was distributed between water and chloroform. The aqueous layer was further extracted with chloroform, and the organic layers were combined and dried over anhydrous magnesium sulfate. The desiccant was removed by filtration, and the filtrate was concentrated under reduced pressure and purified by flash column chromatography (silica gel : Merk Kiesel Gel 60, 230 - 400 mesh, eluent: chloroform/ethanol = 25 : 1) to give 3.0 g of 4-hydroxy-4'-fluoro-2'-methylthiobutyrophenone oxime as a mixture of E and Z isomers.
   m.p. 94.0 - 106.0 °C
(7) A mixture of 2.50 g of 4-hydroxy-4'-fluoro-2'-methylthiobutyrophenone oxime, 5 ml of acetic anhydride and 50 ml of pyridine was refluxed under heating for 28 hours. After cooling, the solvent was distilled away under reduced pressure, and the residue was distributed between 1 N hydrochloric acid and chloroform. The aqueous layer was further extracted with chloroform, and the organic layers were combined, washed with 1 N hydrochloric acid and then with water and dried over anhydrous magnesium sulfate. The desiccant was removed by filtration, and the filtrate was concentrated under reduced pressure and purified by flash column chromatography (silica gel : Merk Kiesel Gel 60, 230 - 400 mesh, eluent: hexane/ethyl acetate = 5 : 1) to give 1.76 g of 3-(3-acetoxypropyl)-6-fluoro-1,2-benzisothiazole.
   m.p. 33.0 - 34.0°C
(8) 1.70 g of 3-(3-acetoxypropyl)-6-fluoro-1,2-benzisothiazole was dissolved in 17 ml of ethanol, and 6.7 ml of 2 N aqueous potassium hydroxide was added under cooling on ice, and the mixture was stirred for 1 hour at the same temperature. The reaction solution was neutralized with 1 N hydrochloric acid, and the solvent was distilled away under reduced pressure and distributed between water and chloroform. The aqueous layer was further extracted with chloroform, and the organic layers were combined and dried over anhydrous magnesium sulfate. The desiccant was removed by filtration, and the filtrate was concentrated under reduced pressure and purified by flash column chromatography (silica gel : Merk Kiesel Gel 60, 230 - 400 mesh, eluent: hexane/ethyl acetate = 2 : 1 - 3 : 2) to give 1.35 g of 3-(3-hydroxypropyl)-6-fluoro-1,2-benzisothiazole.
   m.p. 57.0 - 58.0°C
(9) 0.40 g of 3-(3-hydroxypropyl)-6-fluoro-1,2-benzisothiazole and 0.40 ml of triethylamine were dissolved in 6 ml of dichloromethane, and 2 ml of a dichloromethane solution containing 0.18 ml of methanesulfonyl chloride was added dropwise under cooling at -10 °C. After dropwise addition, the reaction mixture was stirred at -10 °C for 1 hour and then at room temperature for 1 hour. After ice water was added to the reaction solution, the solution was extracted with chloroform, and the organic layer was dried over anhydrous magnesium sulfate. The desiccant was removed by filtration, and the filtrate was concentrated under reduced pressure to give 0.55 g of 3-(3-methanesulfonyloxypropyl)-6-fluoro-1,2-benzisothiazole. This compound was used in the subsequent reaction without further purification.

A mixture of 0.55 g of 3-(3-methanesulfonyloxypropyl)-6-fluoro-1,2-benzisothiazole, 0.375 g of 2-amino-4-(4-fluorophenyl)-5-(pyrrolidin-3-yl)thiazole, 0.5 ml of diisopropylethylamine and 4 ml of N,N-dimethylformamide was stirred at room temperature for 6 days. The reaction solution was distributed between water and ethyl acetate, and the aqueous layer was further extracted with ethyl acetate. The organic layers were combined, washed with water and then with saturated aqueous sodium chloride and dried over anhydrous magnesium sulfate. The desiccant was removed by filtration, and the filtrate was concentrated under reduced pressure and purified by flash column chromatography (silica gel : Chromatorex NHDM1020 (Fuji-Davison Chemical Ltd.), eluent: chloroform) and recrystallized from ethanol/hexane to give 0.376 g of 2-amino-4-(4-fluorophenyl)-5-[1-[3-(6-fluoro-1,2-benzisothiazol-3-yl)propyl]pyrrolidin-3-yl]thiazole.
m.p. 132.0 - 133.0°C

The structures and physical data of this compound and compounds obtained in the same manner are shown in Table E.

### Example 8

### Synthesis of 2-amino-5-(2-chloroethyl)-4-(4-fluorophenyl)thiazole hydrobromide

10.03 g of 4-chloro-4'-fluorobutyrophenone was dissolved in 40 ml of acetic acid, and one drop of 47 % aqueous hydrobromic acid was added, and 10 ml of acetic acid solution containing 8.07 g of bromine was added dropwise for 30 minutes. This reaction mixture was stirred at room temperature for 1.5 hours, and the acetic acid was distilled away under reduced pressure.

To this residue were added 50 ml ethanol and 3.81 g of thiourea, and the mixture was refluxed under heating for 5 hours. The crystal obtained by concentrating the reaction mixture under reduced pressure was recrystallized from ethanol to give 11.62 g of 2-amino-5-(2-chloroethyl)-4-(4-fluorophenyl)thiazole hydrobromide.
m.p. 185.0 - 187.0°C

The structures and physical data of this compound and compounds obtained in the same manner are shown in Table I.

The free base was obtained by neutralizing with aqueous sodium hydroxide or saturated aqueous sodium hydrogen carbonate. The product was purified as necessary by silica gel column chromatography (eluent: hexane/ethyl acetate = 50 : 1 - 5 : 1).

### Example 9

### Synthesis of 5-(2-chloroethyl)-4-(4-fluorophenyl)thiazole hydrochloride

10.03 g of 4-chloro-4'-fluorobutyrophenone was dissolved in 50 ml of carbon tetrachloride, and 8.68 g of bromine was added dropwise for 15 minutes. This reaction mixture was stirred at room temperature for 1.5 hours and concentrated under reduced pressure.

This residue was added to a suspension of 2.36 g of formamide and 2.53 g of phosphorous pentasulfide in 100 ml of dioxane previously stirred under heating at 100 °C for 1.5 hours, and the mixture was stirred under heating at 100°C for 5 hours. The reaction mixture was concentrated under reduced pressure, neutralized with saturated aqueous sodium hydrogen carbonate and extracted with ethyl acetate. The extract was washed with saturated aqueous sodium hydrogen carbonate and then with saturated aqueous sodium chloride, and dried over anhydrous sodium sulfate. The desiccant was removed by filtration, and the filtrate was concentrated under reduced pressure and purified by flash column chromatography (silica gel: Wako Gel C200, eluent: hexane/ethyl acetate = 10 : 1). This free base was treated with 4 N hydrogen chloride/ethyl acetate and recrystallized from isopropanol to give 3.10 g of 5-(2-chloroethyl)-4-(4-fluorophenyl)thiazole hydrochloride.
m.p. 114.5 - 116.5°C

The structures and physical data of this compound are shown in Table I.

### Example 10

### Synthesis of 2-amino-5-(2-bromoethyl)-4-(4-fluorophenyl)thiazole hydrobromide

A mixture of 10.1 g of 2-amino-5-(2-chloroethyl)-4-(4-fluorophenyl)thiazole hydrobromide and 100 ml of 47 % aqueous hydrobromic acid was refluxed under heating for 2.5 hours. After cooling to room temperature, the solvent was distilled away under reduced pressure, and ether and a small amount of ethanol were added to the residue, and the crystal formed was collected by filtration and recrystallized from ethanol to give 9.4 g of 2-amino-5-(2-bromoethyl)-4-(4-fluorophenyl)thiazole hydrobromide.
m.p. 162.0 - 164.0°C

The structures and physical data of this compound and compounds obtained in the same manner are shown in Table I.

The conversion of the chlorine atom into an iodine atom was carried out in the same manner as above using 57 % aqueous hydroiodic acid in place of 47 % aqueous hydrobromic acid. The free base was obtained by neutralization with aqueous sodium hydroxide or saturated aqueous sodium hydrogen carbonate.

### Example 11

### Synthesis of 2-(4-fluorophenyl)-2-(1,2,3,6-tetrahydro-4-pyridinyl)-1, 3-dioxolane

(1) 5.00 g of 4-(4-fluorobenzoyl)pyridine, 2.40 g of ethylene glycol and 5.70 g of p-toluenesulfonic acid·1H₂O in 50 ml of benzene were refluxed under heating for 22 hours while formed water was removed by azeotropic distillation. The reaction solution was washed with 2 N aqueous sodium hydroxide and then with saturated aqueous sodium chloride. The organic layer was dried over anhydrous sodium sulfate, then the desiccant was removed by filtration, and the solvent was evaporated under reduced pressure.
   This residue was dissolved in 30 ml of acetonitrile, and 6.94 g of methyl iodide was added, and the mixture was stirred for 3 hours and the reaction solution was concentrated under reduced pressure.
   This residue was dissolved in 45 ml of methanol, and 2.30 g of sodium borohydride was added with small portions over a period of 30 minutes, and the mixture was further stirred at room temperature for 1 hour. The solvent was distilled away under reduced pressure and the residue was distributed between dichloromethane and water. The aqueous layer was further extracted with dichloromethane, and the organic layers were combined and dried over anhydrous sodium sulfate. The desiccant was removed by filtration, and the filtrate was concentrated under reduced pressure and purified by flash column chromatography (silica gel : Wako Gel C200, eluent: chloroform/ethanol = 15 : 1) to give 5.57 g of 2-(4-fluorophenyl)-2-(1,2,3,6-tetrahydro-1-methyl-4-pyridinyl)-1,3-dioxolane.
   NMR (CDCl₃) δ (ppm): 2.05 - 2.15 (2H, m), 2.33 (3H, s), 2.47 (2H, t, J = 5.8 Hz), 2.95 - 3.00 (2H, m), 3.90 - 4.06 (4H, m), 5.83 - 5.89 (1H, m), 6.95 - 7.05 (2H, m), 7.43 - 7.51 (2H, m)
   MS m/e: 264 (M⁺+1, 100 %)
(2) 2.50 g of 2-(4-fluorophenyl)-2-(1,2,3,6-tetrahydro-1-methyl-4-pyridinyl)-1,3-dioxolane and 1.20 g of diisopropylethylamine were dissolved in 12.5 ml of benzene, and 3.20 g of ethyl chlorocarbonate was added at room temperature and the mixture was refluxed under heating for 30 minutes. The reaction mixture was washed with 0.5 N aqueous sodium hydroxide, water and saturated aqueous sodium chloride in order and dried over anhydrous magnesium sulfate. The desiccant was removed by filtration, and the filtrate was concentrated under reduced pressure and purified by flash column chromatography (silica gel : Wako Gel C200, eluent: hexane/ethyl acetate = 5 : 1) to give 2.92 g of 2-(4-fluorophenyl)-2-(1,2,3,6-tetrahydro-1-ethoxycarbonyl-4-pyridinyl)-1,3-dioxolane.
   NMR (CDCl₃) δ (ppm): 1.25 (3H, t, J = 7.1 Hz), 2.02 - 2.13 (2H, m), 3.49 (2H, t, J = 5.7 Hz), 3.89 - 4.05 (6H, m), 4.13 (2H, q, J = 7.1 Hz), 5.77 - 5.84 (1H, m), 6.95 - 7.07 (2H, m), 7.38 - 7.49 (2H, m)
   MS m/e: 322 (M⁺+1, 100 %)
(3) A mixture of 1.40 g of 2-(4-fluorophenyl)-2-(1,2,3,6-tetrahydro-1-ethoxycarbonyl-4-pyridinyl)-1,3-dioxolane and 4.10 g of barium hydroxide·8H₂O was refluxed in a mixed solvent of 70 ml of methanol and 70 ml of water under heating for 27 hours. The reaction solvent was distilled away under reduced pressure and the residue was diluted with chloroform and washed 5 times with saturated aqueous ammonium chloride and then with saturated aqueous sodium chloride. The organic layer was dried over anhydrous sodium sulfate. The desiccant was removed by filtration, and the filtrate was purified by flash column chromatography (silica gel: Chromatorex NHDM1020 (Fuji-Davison Chemical Ltd.), eluent: hexane/ethyl acetate = 4 : 1 - 1 : 1) to give 556 mg of 2-(4-fluorophenyl)-2-(1,2,3,6-tetrahydro-4-pyridinyl)-1,3-dioxolane.
   m.p. 65.5 - 66.5°C

### Example 12

### Synthesis of 4-[1-(4-fluorophenyl)ethylen-1-yl]piperidine hydrochloride

1.89 g of N-t-butoxycarbonyl-4-(4-fluorobenzoyl)piperidine was dissolved in 10 ml of diethyl ether, and 6.2 ml of diethyl ether solution containing 1 M methylmagnesium iodide was added under cooling on ice. The mixture was stirred at room temperature for 30 minutes, and saturated aqueous ammonium chloride was added and the mixture was extracted with ethyl acetate. The organic layer was washed with 5 % aqueous potassium hydrogen sulfate and then with saturated aqueous sodium chloride, and dried over anhydrous magnesium sulfate. The desiccant was removed by filtration, and the solvent was distilled away under reduced pressure. The resulting residue was dissolved in 5 ml of dichloromethane, and 10 ml of trifluoroacetic acid was added, and the mixture was stirred at room temperature for 13 hours. The reaction solution was concentrated under reduced pressure and subjected to azeotropic distillation 3 times with toluene. The residue was dissolved in 5 ml of dichloromethane, and 2 ml of 4 N hydrogen chloride/dioxane was added and the reaction solution was concentrated under reduced pressure. The residue was recrystallized from isopropanol to give 1.0 g of 4-[1-(4-fluorophenyl)ethylen-1-yl]piperidine hydrochloride.
m.p. 215.0 - 216.5°C

### Example 13

### Synthesis of 4-(4-fluorobenzylidene)piperidine hydrochloride

(1) A solution of 59.78 g of N-t-butoxycarbonyl-4-piperidone and 81.25 g of diethyl 4-fluorobenzylphosphonate in 150 ml of tetrahydrofuran was added dropwise to a stirred suspension of 13.20 g of 60 % sodium hydride (in oil) containing 1.65 g of 15-crown-5 ether in 650 ml of tetrahydrofuran, under cooling on ice for 20 minutes. The mixture was stirred for one day at room temperature, and saturated aqueous sodium hydrogen carbonate was added carefully, and the mixture was extracted with ethyl acetate. The extract was washed with saturated aqueous sodium hydrogen carbonate and then with saturated aqueous sodium chloride and dried over anhydrous sodium sulfate. The desiccant was removed by filtration, and the filtrate was concentrated under reduced pressure and purified by flash column chromatography (silica gel : Wako Gel C200, eluent: hexane/ethyl acetate = 20 : 1) to give 55.23 g of N-t-butoxycarbonyl-4-(4-fluorobenzylidene)piperidine. The resulting oily substance was crystallized by allowing to stand overnight at room temperature.
   m.p. 69.0 - 70.0°C
(2) 475 ml of a 4 N hydrogen chloride/dioxane cooled on ice was added to 55.00 g of N-t-butoxycarbonyl-4-(4-fluorobenzylidene)piperidine, and the mixture was stirred at room temperature for 2 hours. The crystal obtained by concentrating the reaction solution under reduced pressure was recrystallized from isopropanol to give 40.72 g of 4-(4-fluorobenzylidene)piperidine hydrochloride.
   m.p. 184.0 - 185.5°C

The structures and physical data of this compound and compounds obtained in the same manner are shown in Table J.

In the case of synthesis of α-alkylbenzylidenepiperidine derivatives, the base used was lithium diisopropylamide in place of sodium hydride (containing 15-crown-5 ether), and the reaction temperature was raised to room temperature after dropwise addition at -50°C.

### Example 14

### Synthesis of 2-amino-4-(4-fluorophenyl)-5-[2-[4-(4-fluorobenzoyl)piperidin-1-yl]ethyl]thiazole

1.01 g of 2-amino-5-(2-chloroethyl)-4-(4-fluorophenyl)thiazole hydrobromide, 1.14 g of 4-(4-fluorobenzoyl)piperidine p-toluenesulfonate and 2.1 ml of diisopropylethylamine were stirred in 3 ml of methanol at 65°C for 1.5 days. This reaction mixture was poured into saturated aqueous sodium hydrogen carbonate, extracted with ethyl acetate, and washed with water and saturated aqueous sodium chloride. The extract was dried over anhydrous sodium sulfate, and the desiccant was removed by filtration, and the filtrate was concentrated under reduced pressure and purified by flash column chromatography (silica gel: Chromatorex NHDM1020 (Fuji-Davison Chemical Ltd.), eluent: hexane/ethyl acetate = 4 : 1 - 1 : 1) and recrystallized from ethyl acetate to give 0.49 g of 2-amino-4-(4-fluorophenyl)-5-[2-[4-(4-fluorobenzoyl)piperidin-1-yl]ethyl]thiazole.
m.p. 203.5 - 205.0°C

The structures and physical data of this compound and compounds obtained in the same manner are shown in Tables K and L.

### Example 15

### Synthesis of 2-amino-4-(4-fluorophenyl)-5-[2-[4-(4-fluorobenzoyl)piperidin-1-yl]ethyl]thiazole

(1) A mixture of 1.26 g of 2-amino-5-(2-bromoethyl)-4-(4-fluorophenyl)thiazole hydrobromide, 2.20 g of 4-[2-(4-fluorophenyl)-1,3-dioxolan-2-yl]piperidine, 3 ml diisopropylethylamine and 3 ml of methanol was refluxed under heating for 12 hours. The reaction solution was cooled to room temperature, and chloroform and saturated aqueous sodium hydrogen carbonate were added. The aqueous layer was further extracted with chloroform. The organic layers were combined, dried over anhydrous magnesium sulfate, concentrated under reduced pressure, purified by silica gel column chromatography (silica gel : Merk Kiesel Gel 60, 230 - 400 mesh, eluent: acetone/hexane = 2 : 3), and recrystallized from ethanol/hexane to give 1.68 g of 2-amino-4-(4-fluorophenyl)-5-[2-[4-[2-(4-fluorophenyl)-1,3-dioxolan-2-yl]piperidine-1-yl]ethyl]thiazole.
   m.p. 127.0 - 129.0°C
(2) 2-amino-4-(4-fluorophenyl)-5-[2-[4-[2-(4-fluorophenyl)-1,3-dioxolan-2-yl]piperidin-1-yl]ethyl]thiazole was subjected to the same acid treatment as in Example 5 to give 2-amino-4-(4-fluorophenyl)-5-[2-[4-(4-fluorobenzoyl)piperidin-1-yl]ethyl]thiazole.
   m.p. 203.5 - 205.0°C

The structures and physical data of this compound and compounds obtained in the same manner are shown in Table K.

### Example 16

### Synthesis of 2-amino-5-[2-[4-(4-fluorobenzylidene)piperidin-1-yl]ethyl]-4-phenylthiazole

959 mg of 2-amino-5-(2-chloroethyl)-4-phenylthiazole hydrobromide, 717 mg of 4-(4-fluorobenzylidene)piperidine hydrochloride and 1.6 ml of diisopropylethylamine were stirred in 2 ml of methanol at 80 °C for 3 days. This reaction mixture was poured into saturated aqueous sodium hydrogen carbonate, extracted with ethyl acetate, and washed with water and saturated aqueous sodium chloride. The extract was dried over anhydrous sodium sulfate, then the desiccant was removed by filtration, and the filtrate was concentrated under reduced pressure and purified by flash column chromatography (silica gel: Chromatorex NHDM1020 (Fuji-Davison Chemical Ltd.), eluent: hexane/ethyl acetate = 2 : 1 - 1 : 1) and recrystallized from ethyl acetate to give 769 mg of 2-amino-5-[2-[4-(4-fluorobenzylidene)piperidin-1-yl]ethyl]-4-phenylthiazole.
m.p. 151.5 - 153.0°C

The structures and physical data of this compound and compounds obtained in the same manner are shown in Table L.

### Example 17

### Synthesis of 2-amino-5-(4-fluorophenyl)-4-[1-(2-phenylethyl)piperidin-4-yl]thiazole

2.01 g of 4-[2-(4-fluorophenyl)-1-oxoethyl]-1-(2-phenylethyl)piperidine hydrochloride was dissolved in a mixed solvent of acetic acid-chloroform (10 ml-5 ml), and 0.3 ml of bromine was added dropwise for 10 minutes. This reaction mixture was stirred at room temperature for 1 hour, and the solvent was distilled away under reduced pressure.

To this residue were added 20 ml of ethanol and 511 mg of thiourea, and the mixture was refluxed under heating for 2 hours. The reaction mixture was concentrated under reduced pressure and distributed between 0.5 N aqueous sodium hydroxide and dichloromethane. The aqueous layer was further extracted twice with dichloromethane, and the extracts were combined and dried over anhydrous sodium sulfate. The desiccant was removed by filtration, and the filtrate was concentrated under reduced pressure, and the residue was recrystallized from methanol to give 1.62 g of 2-amino-5-(4-fluorophenyl)-4-[1-(2-phenylethyl)piperidin-4-yl]thiazole.
m.p. 205.5 - 207.5°C

The structure and physical data of this compound are shown in Table B.

### Example 18

### Synthesis of 2-amino-4-(4-fluorophenyl)-5-[1-[4-(4-hydroxyphenyl)-4-oxobutyl]pyrrolidin-3-yl]thiazole dihydrobromide

47 % aqueous hydrobromic acid was added to 50 mg of 2-amino-4-(4-fluorophenyl)-5-[1-[4-(4-methoxyphenyl)-4-oxobutyl]pyrrolidin-3-yl]thiazole, and the mixture was refluxed under heating for 3 hours. The reaction mixture was concentrated under reduced pressure and recrystallized from isopropanol to give 56.0 mg of 2-amino-4-(4-fluorophenyl)-5-[1-[4-(4-hydroxyphenyl)-4-oxobutyl]pyrrolidin-3-yl]thiazole dihydrobromide.
m.p. 173.0 - 175.0°C

The structure and physical data of this compound are shown in Table D.

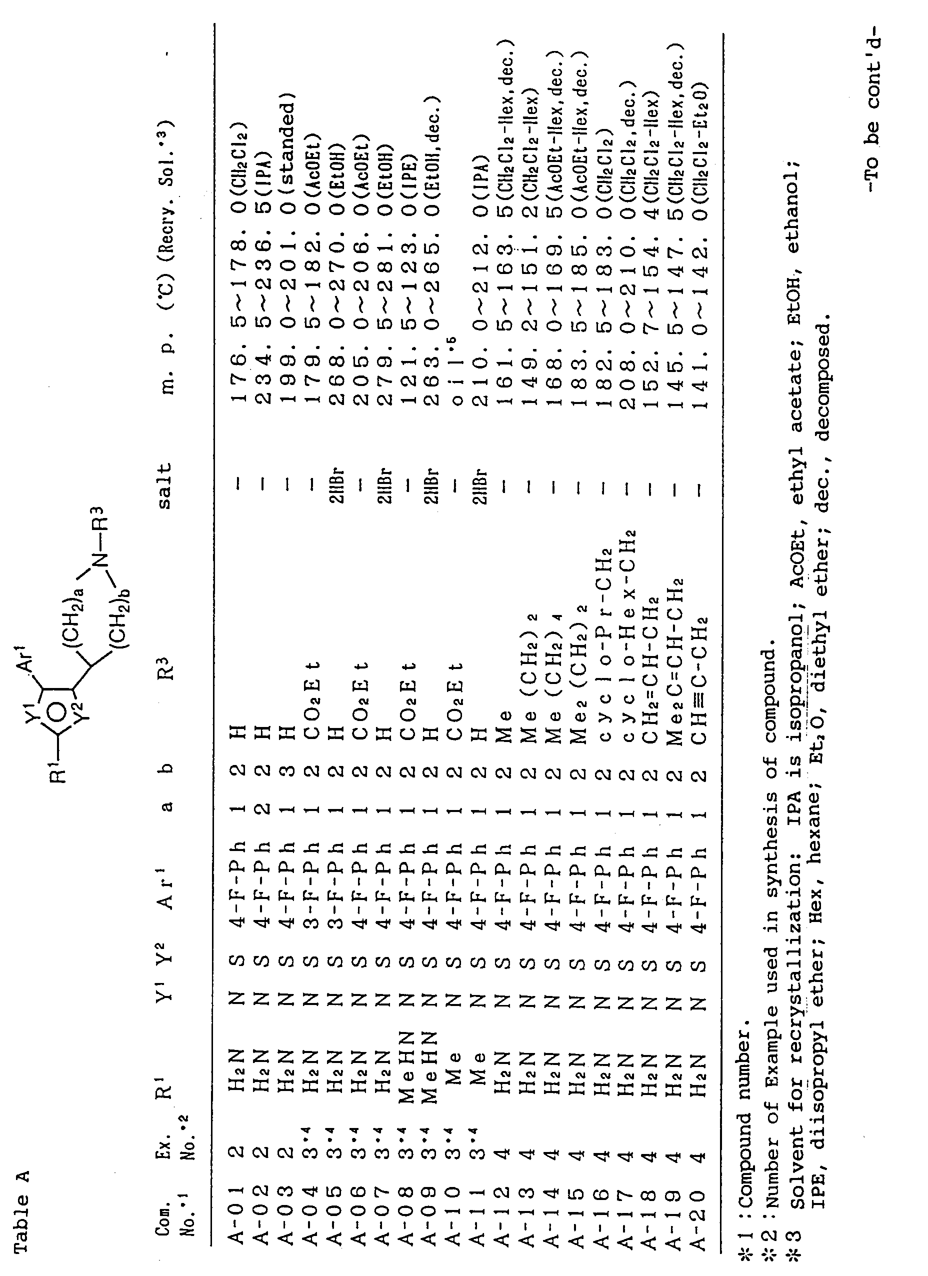

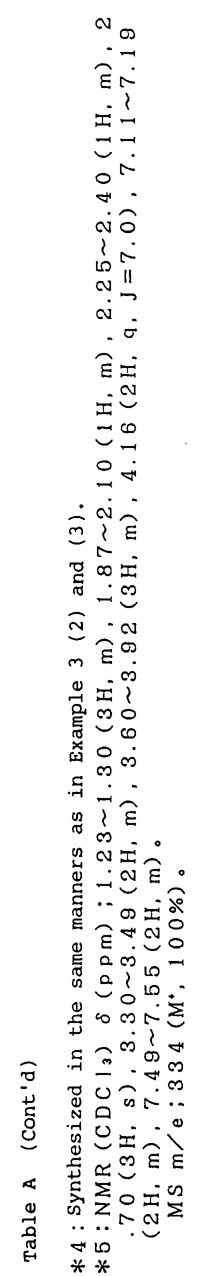

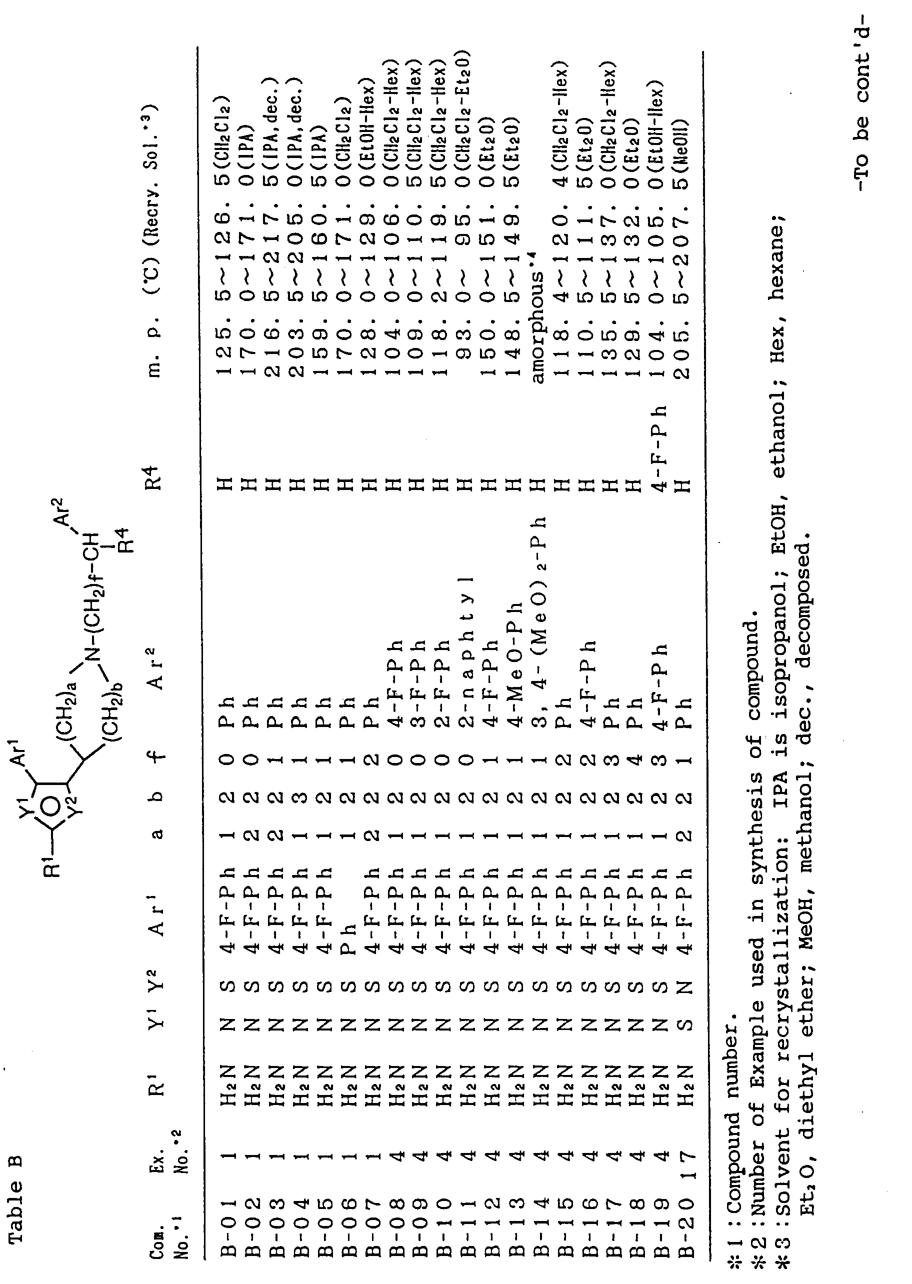

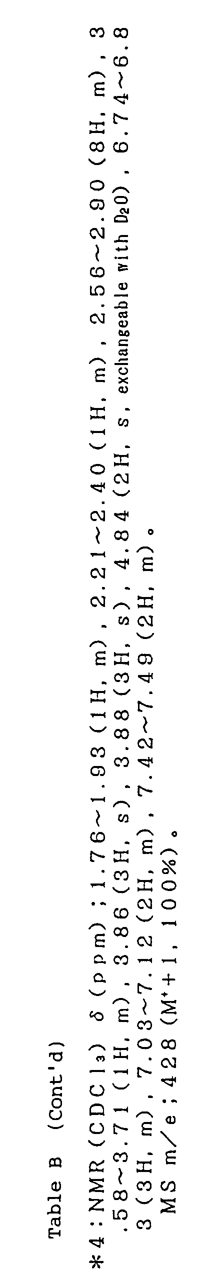

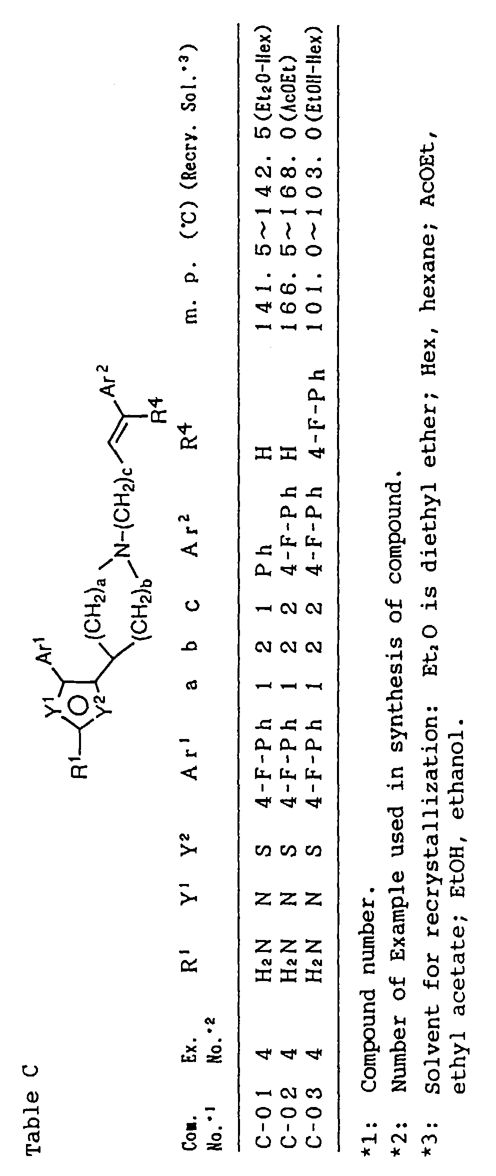

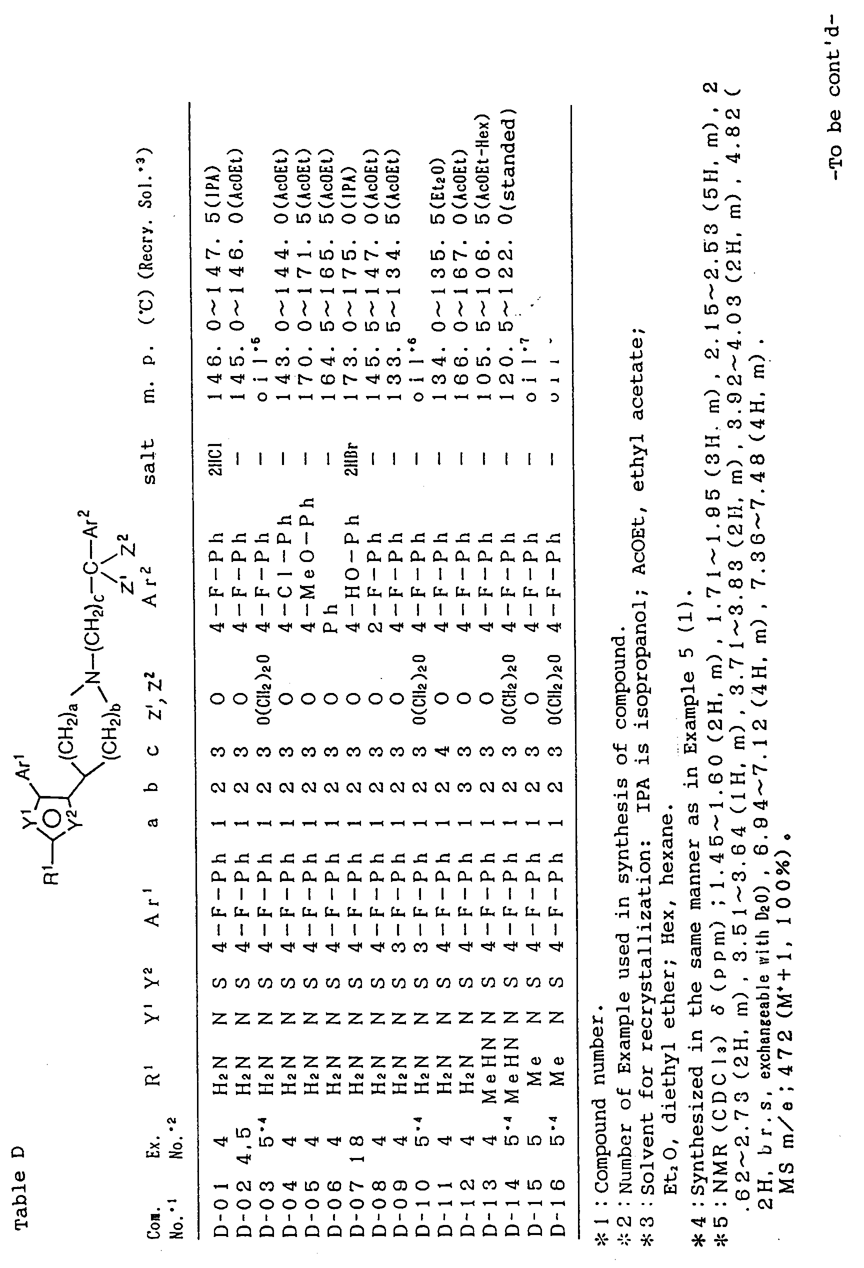

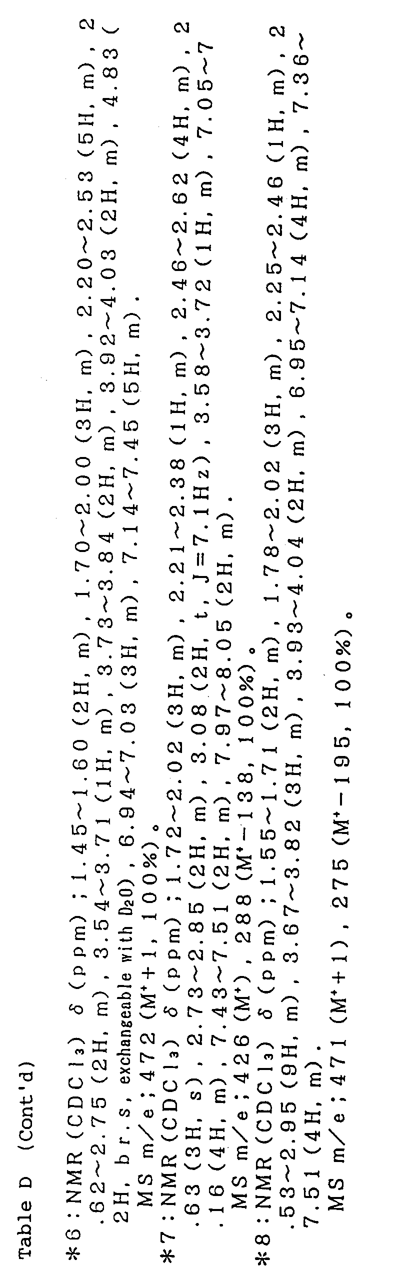

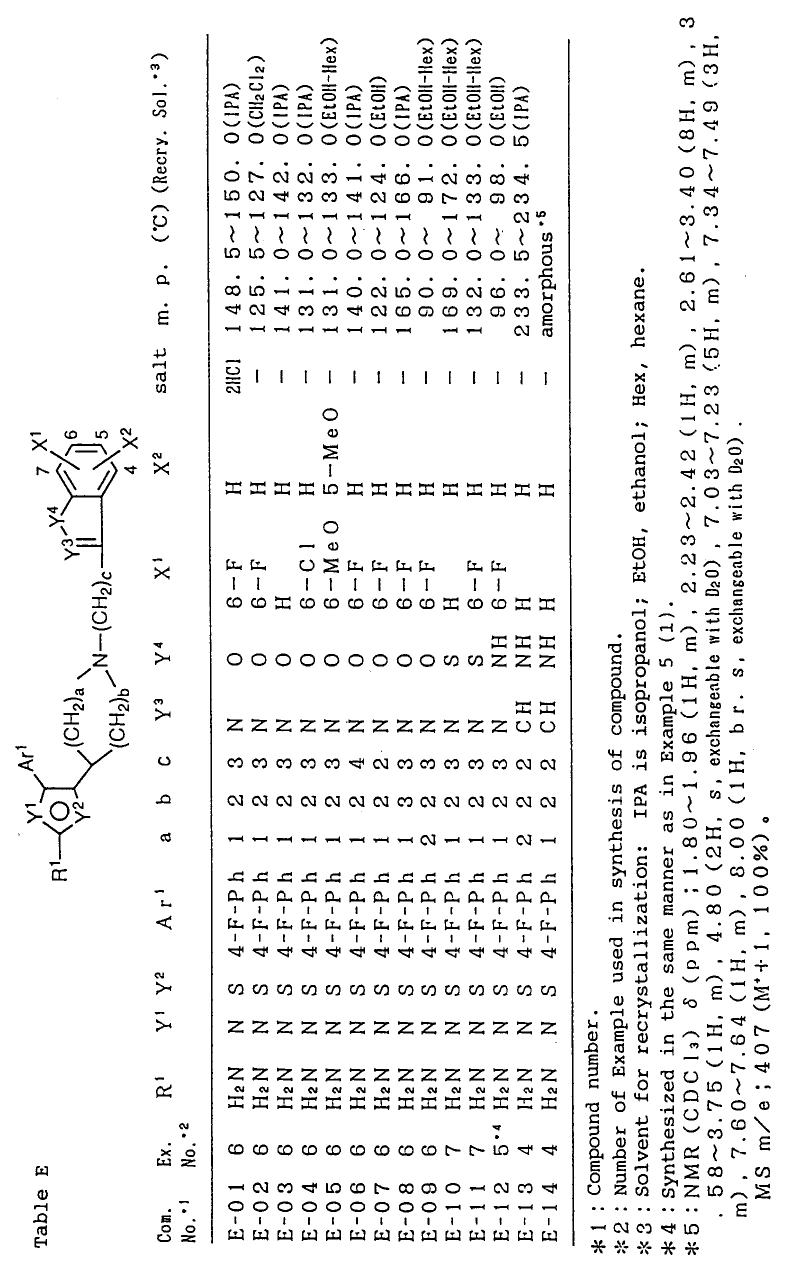

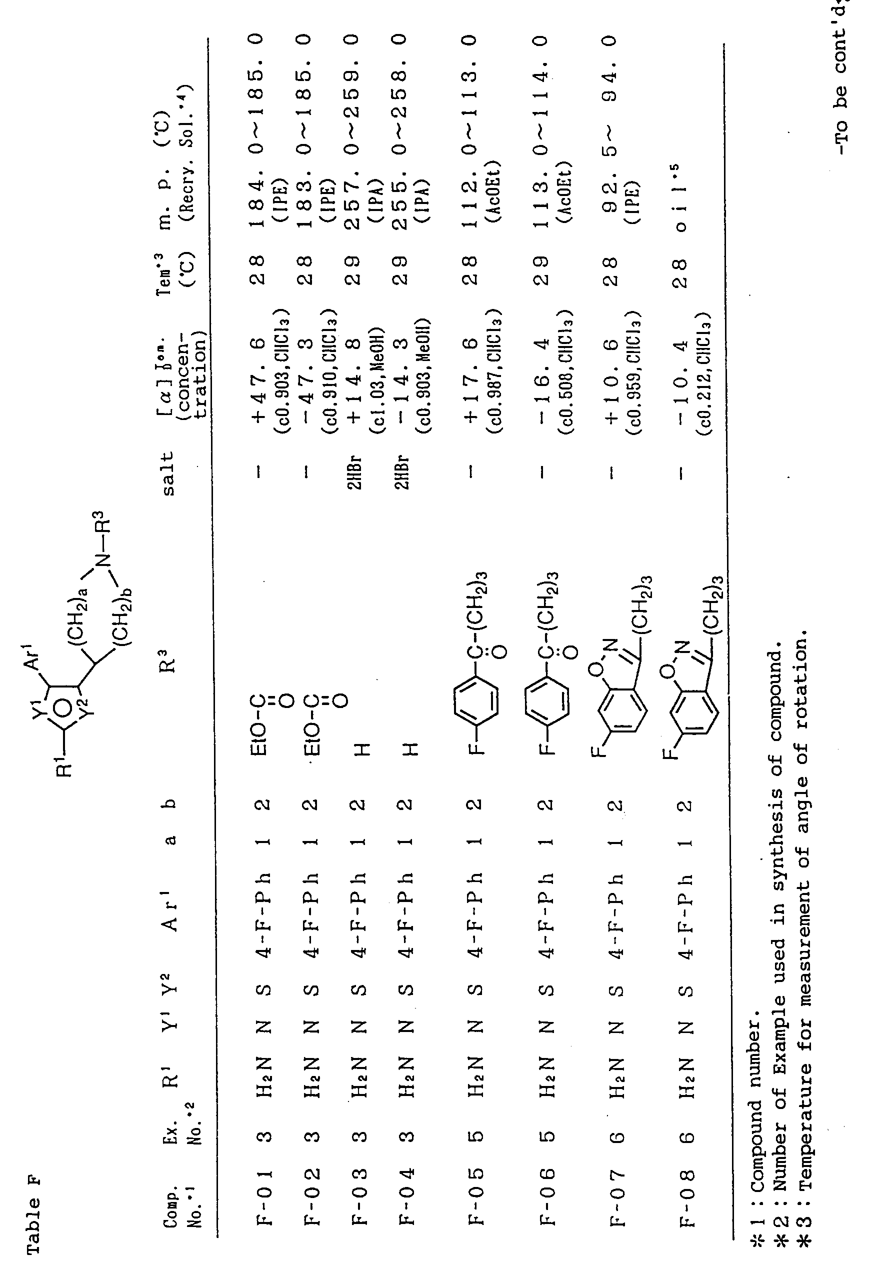

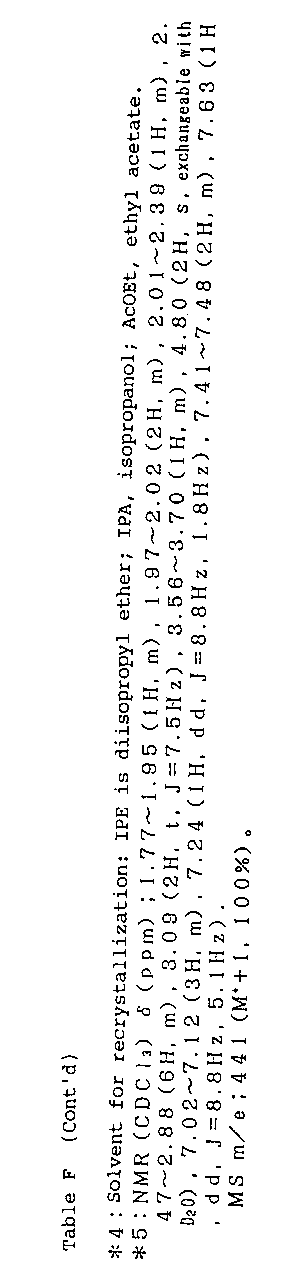

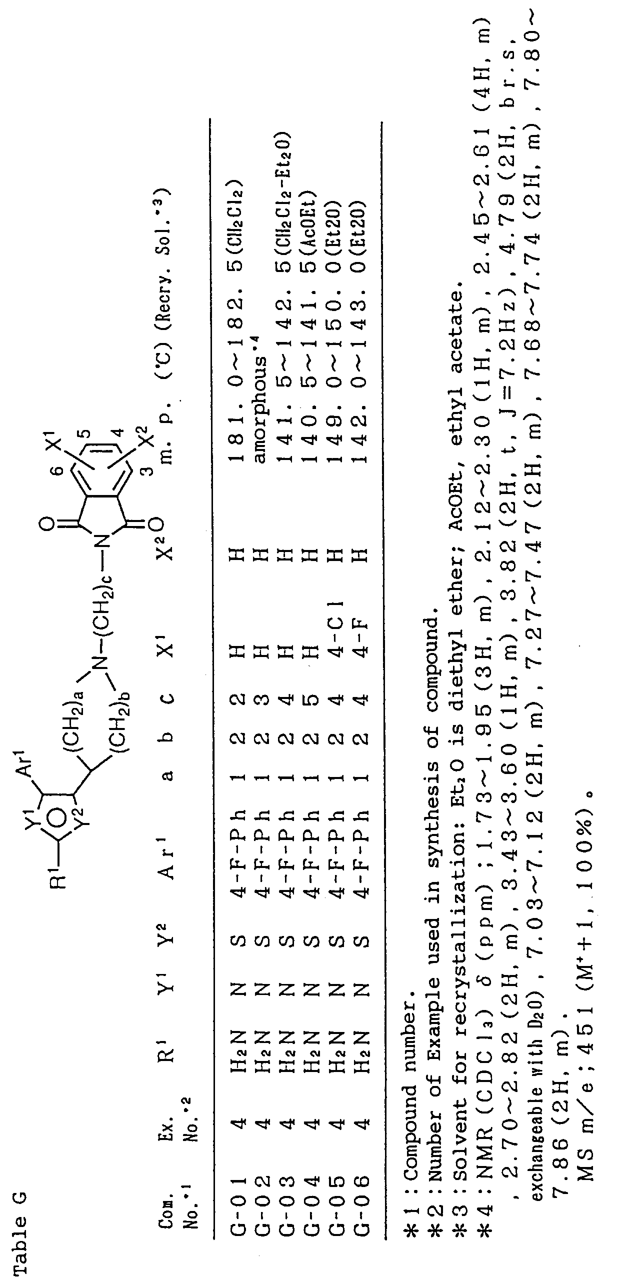

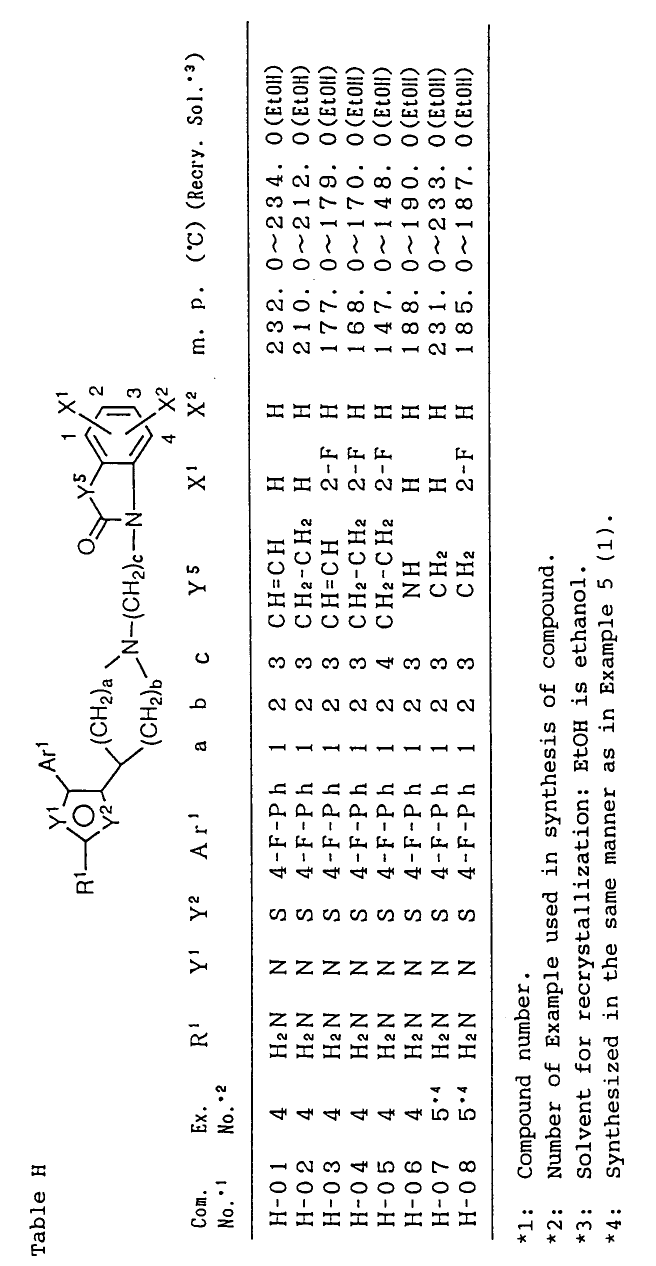

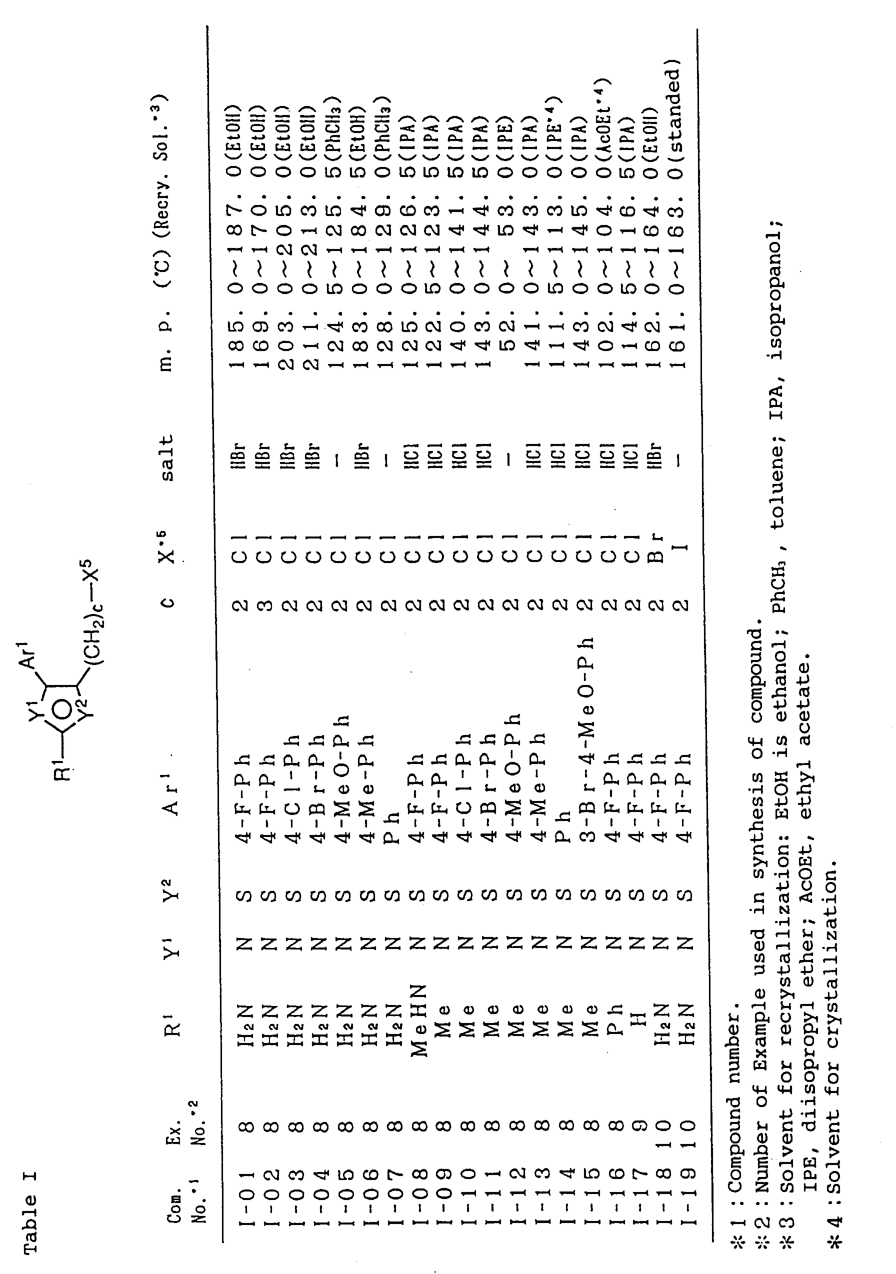

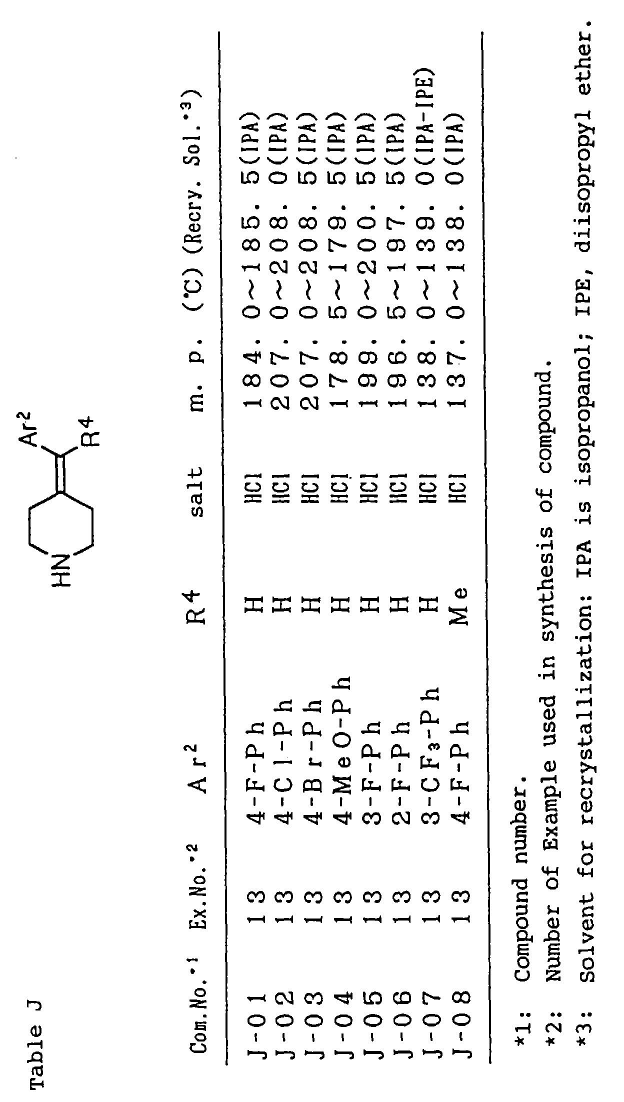

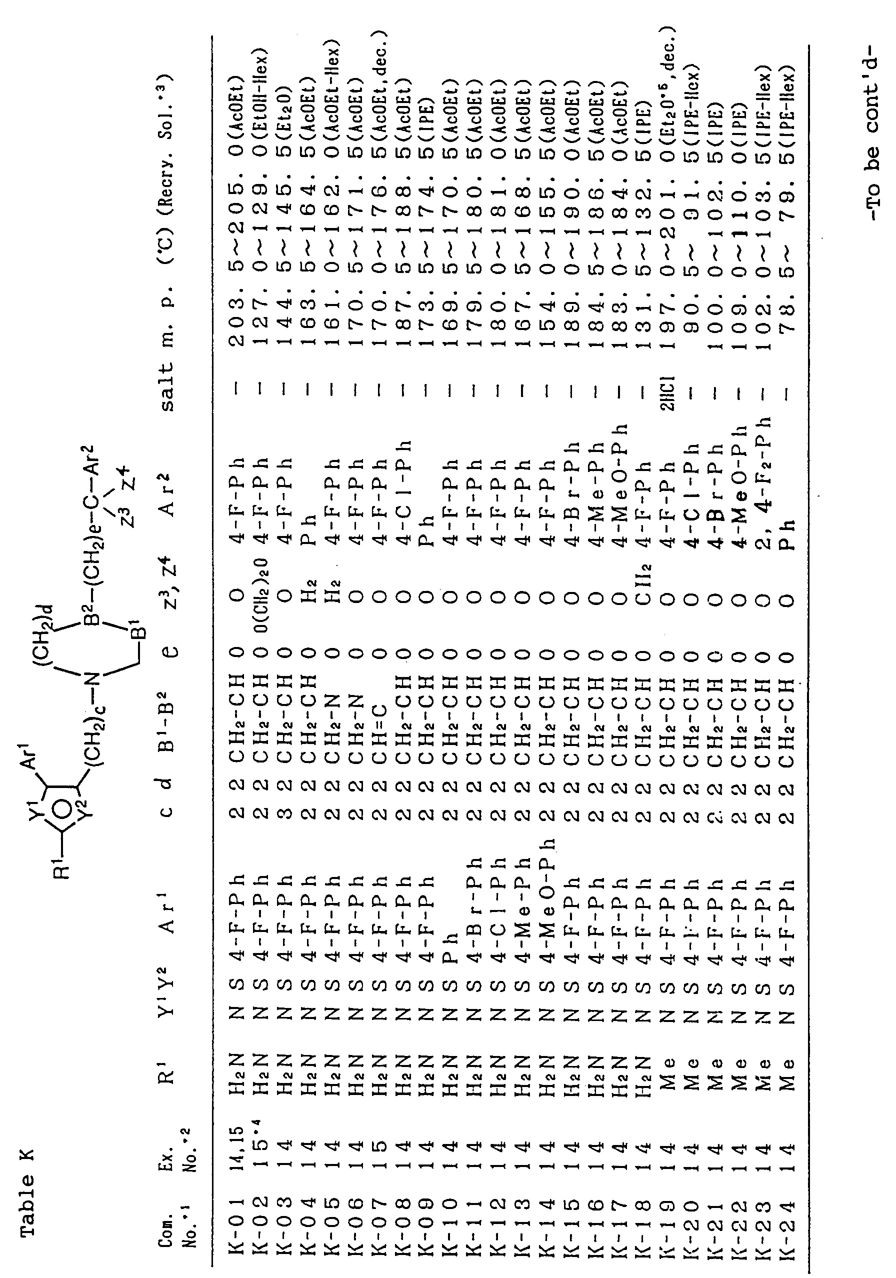

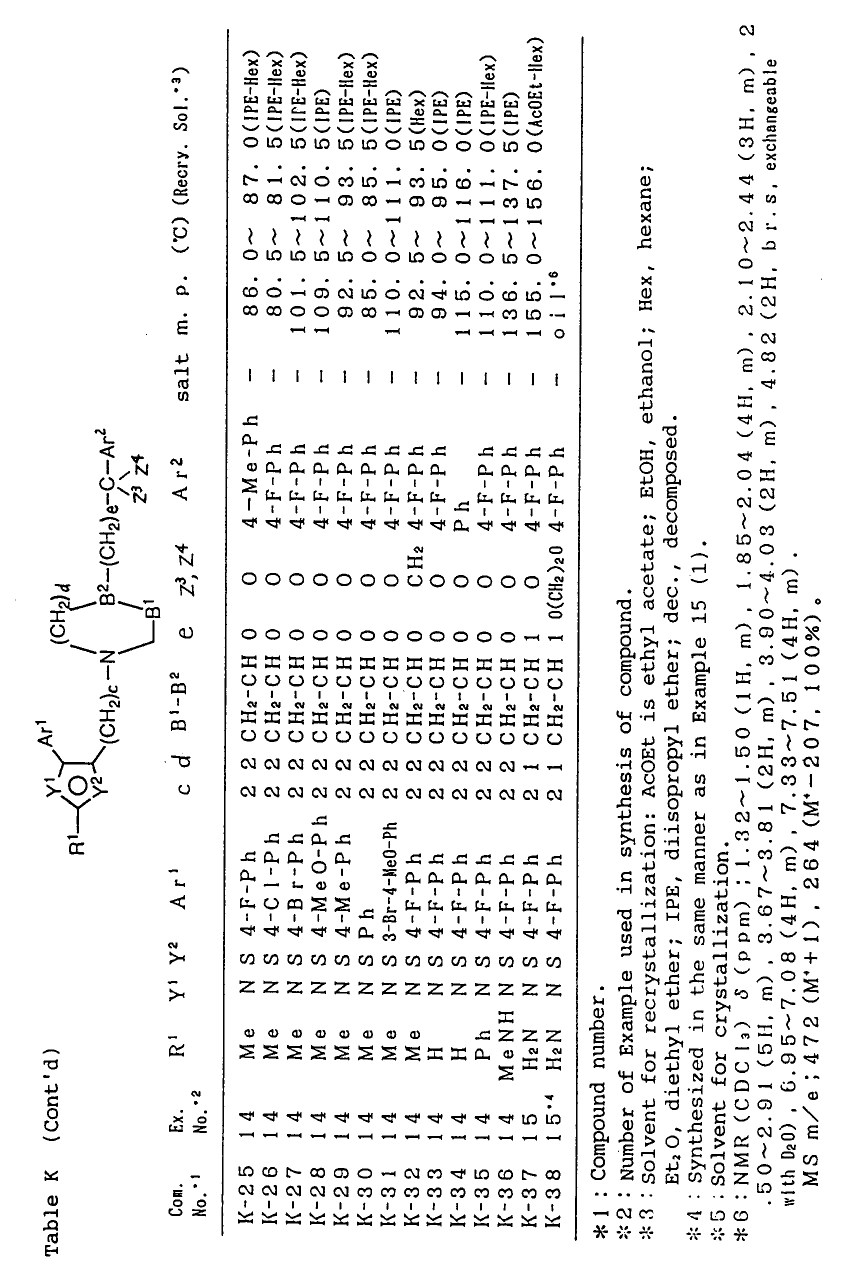

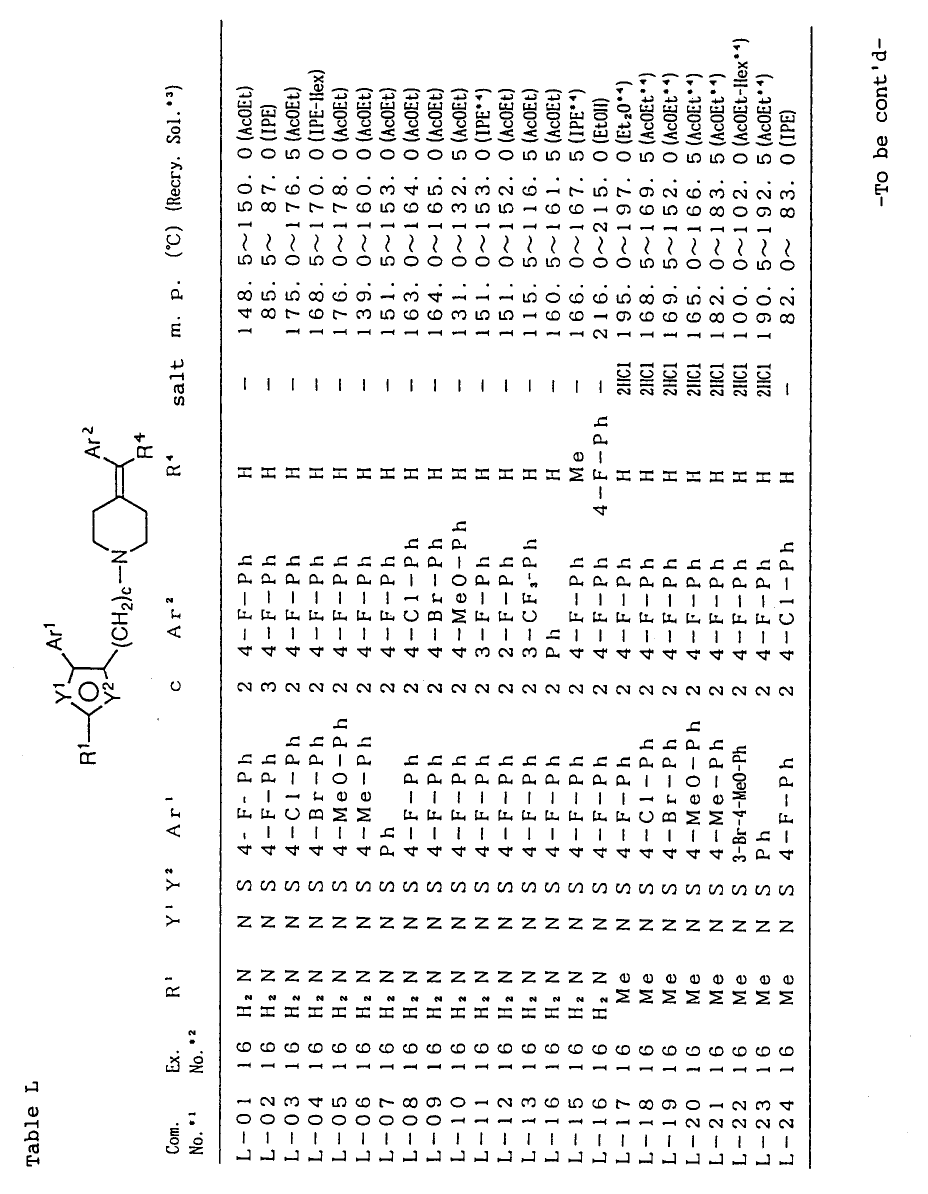

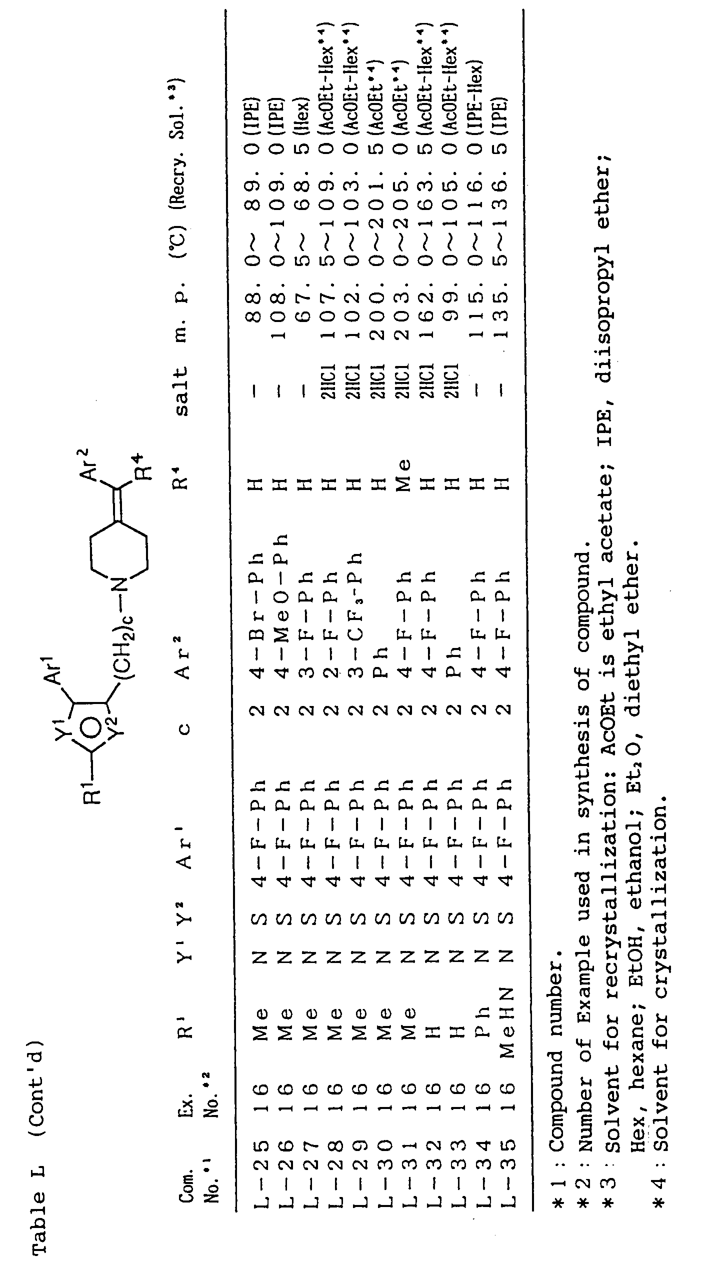

### Test Example: Receptor Binding Experiment

### 1. Dopamine D₄ receptor binding experiment

As a receptor preparation, Chinese hamster ovary (CHO) cell membranes where human D_{4.2} receptor was expressed were used.

As [³H]-labeled ligand, [³H]-spiperone was used.

The binding reaction using the [³H]-labeled ligand was carried out in the following manner as described in Eur. J. Pharmacol., 233, 173 (1993).

Human D_{4.2} receptor binding test: CHO cell membranes where human D_{4.2} receptor was expressed, [³H]-spiperone (0.5 nM) and a test drug were reacted at 27°C for 2 hours in 50 mM Tris-HCl buffer, pH 7.4 containing 5 mM EDTA, 1.5 mM calcium chloride, 5 mM potassium chloride and 120 mM sodium chloride.

After the reaction, the sample was filtered under suction through a glass filter (GF/B), and the radioactivity of the filter paper was determined in a liquid scintillation spectrometer.

The binding of [³H]-spiperone in the presence of 10 µM haloperidol was assumed to be non-specific binding, and the difference between the total binding and the non-specific binding was assumed to be specific binding. By reacting [³H]-spiperone at a predetermined concentration with a test drug at varying concentrations under the conditions described above, an inhibition curve was obtained, and from this inhibition curve, the concentration (IC₅₀) of the test drug at which 50 % of the [³H]-spiperone binding was inhibited was determined. The results are shown in Table M.

### 2. Dopamine D₂ receptor binding experiment

As a receptor preparation, rat striatum membranes were used.

As [³H]-labeled ligand, [³H]-raclopride was used.

The binding reaction using the [³H]-labeled ligand was carried out in the following manner as described in Mol. Pharmacol., 43, 749 (1993).

The preparation of the receptor preparation: Rat striatum was homogenized in 50 mM Tris-HCl buffer, pH 7.4 and centrifuged at 48,000 × g and the residue was washed once with Tris-HCl buffer. The residue was suspended in 50 mM Tris-HCl buffer, pH 7.4 containing 120 mM sodium chloride, 5 mM potassium chloride, 2 mM calcium chloride and 1 mM magnesium chloride and used as the membrane preparation.

Dopamine D₂ receptor binding experiment: The membrane preparation (0.5 mg protein/ml), [³H]-raclopride (1 nM) and a test drug were reacted at 25 °C for 1 hour.

After the reaction, the sample was filtered under suction through a glass filter (GF/B), and the radioactivity of the filter paper was determined in a liquid scintillation spectrometer.

The binding of [³H]-raclopride in the case of reaction in the presence of 10 µM haloperidol was assumed to be non-specific binding, and the difference between the total binding and the non-specific binding was assumed to be specific binding. By reacting [³H]-raclopride at a predetermined concentration with a test drug at varying concentrations under the conditions described above, an inhibition curve was obtained, and from this inhibition curve, the concentration (IC₅₀) of the test drug at which 50 % of the [³H]-raclopride binding was inhibited was determined. The results are shown in Table M.

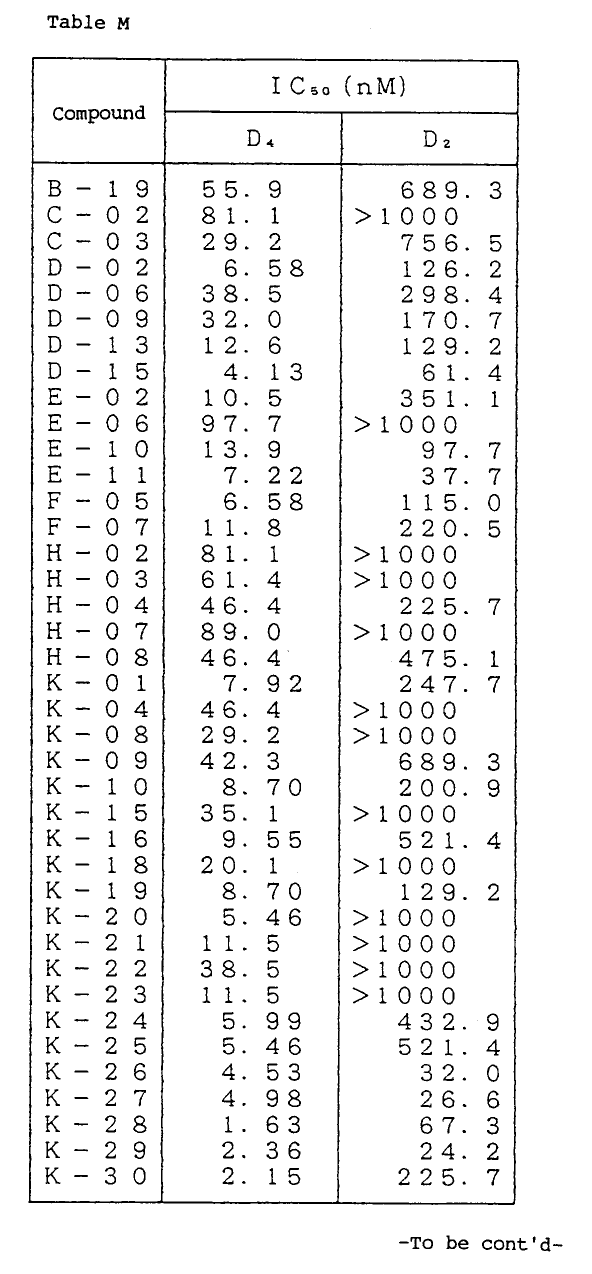

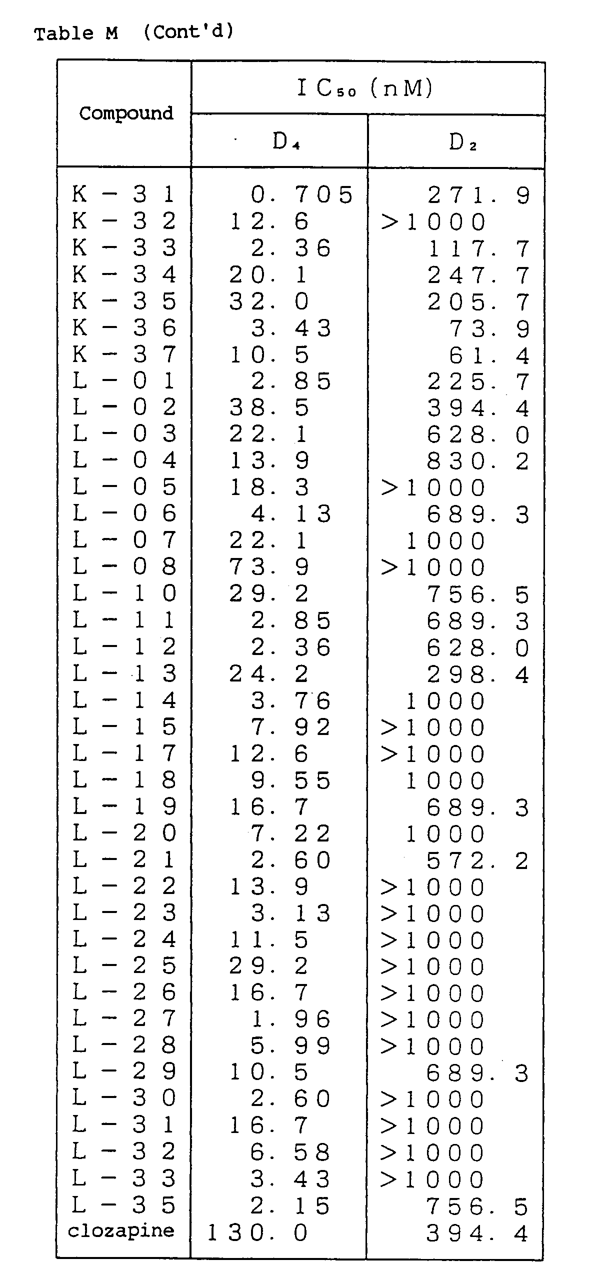

### Industrial Applicability

The compounds of the present invention show an excellent affinity for dopamine D₄ receptor while having a low affinity for dopamine D₂ receptor, thus demonstrating excellent separability.

Therefore, the compounds of the present invention are useful as an agent for prevention and treatment of diseases such as problematic behaviors accompanying schizophrenia, cerebrovascular disease and senile dementia and as an agent not causing extrapyramidal disorder as side effects.

## Claims

1. A thiazole derivative represented by formula (I) 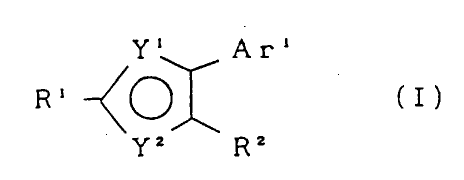 wherein Ar¹ represents a substituted or unsubstituted phenyl group or a thienyl group, Y¹ and Y² are different and each represents a nitrogen atom or a sulfur atom, R¹ represents a hydrogen atom, a C₁ to C₅ alkyl group, a phenyl group, or an amino group unsubstituted or mono- or di-substituted with a C₁ to C₅ alkyl group;
R² represents a group represented by formula (i): 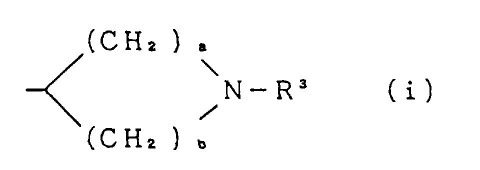 wherein a is an integer of 1 to 3, b is 2 or 3;
R³ represents:
(1) a hydrogen atom, a substituted or unsubstituted C₁ to C₇ alkyl group, a substituted or unsubstituted C₃ to C₇ alkenyl group, a C₃ to C₇ alkynyl group or a C₂ to C₇ alkoxycarbonyl group,
(2) a group represented by the formula: 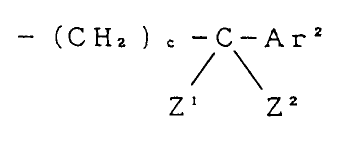 wherein c is an integer of 1 to 5, Ar² represents a substituted or unsubstituted phenyl group or a thienyl group, Z¹ and Z² are the same or different and each represents a C₁ to C₅ alkoxy group or they are combined to form an oxo group or a C₂ to C₃ alkylenedioxy group,
(3) a group represented by the formula: 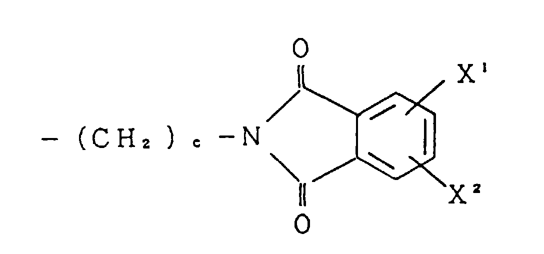 wherein c has the same meaning as defined above, X¹ and X² are the same or different and each represents a hydrogen atom, a halogen atom, a C₁ to C₅ alkoxy group or a hydroxyl group;
(4) a group represented by the formula: 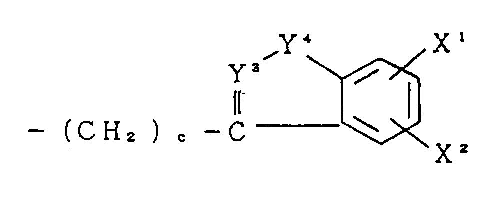 wherein c, X¹ and X² have the same meaning as defined above, Y³ represents N or CH, Y⁴ represents an oxygen atom, a sulfur atom or NH; or
(5) a group represented by the formula: 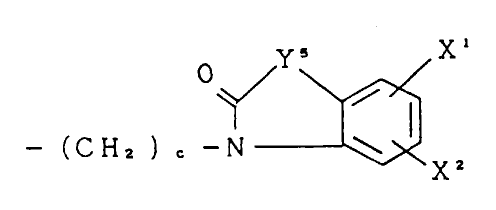 wherein c, X¹ and X² have the same meaning as defined above, and Y⁵ represents CH₂-CH₂, CH=CH, CH₂ or NH;
a group represented by formula (ii): 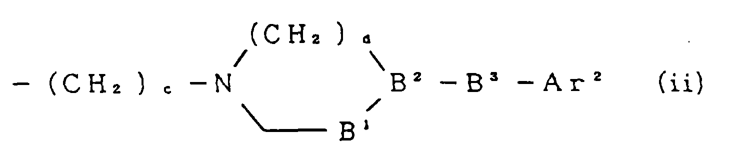 wherein c and Ar² have the same meaning as defined above, d is 1 or 2, B¹-B² represents CH₂-CH, CH=C or CH₂-N, B³ is a group represented by the formula: 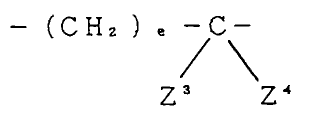 wherein e is 0 or 1, Z³ and Z⁴ both represent a hydrogen atom or are the same or different and each represents a C₁ to C₅ alkoxy group or they are combined to form an oxo group, a methylene group or a C₂ to C₃ alkylenedioxy group, or
a group represented by formula (iii): 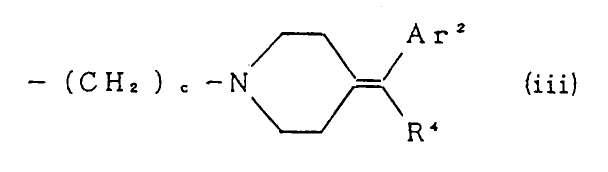 wherein c and Ar² have the same meaning as defined above, R⁴ represents a hydrogen atom, a C₁ to C₅ alkyl group or a substituted or unsubstituted phenyl group, or a pharmaceutically acceptable salt thereof.

2. The compound of formula (I) according to claim 1, wherein Y¹ is a nitrogen atom, and Y² is a sulfur atom.

3. A thiazole derivative represented by formula (I-i-1): 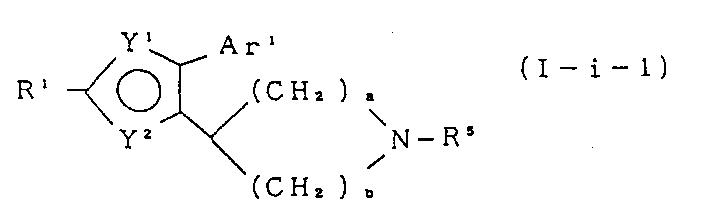 wherein Ar¹, Y¹, Y², R¹, a and b have the same meaning as in claim 1; R⁵ is a hydrogen atom, a substituted or unsubstituted C₁ to C₇ alkyl group, a substituted or unsubstituted C₃ to C₇ alkenyl group, a C₃ to C₇ alkynyl group or a C₂ to C₇ alkoxycarbonyl group, or a pharmaceutically acceptable salt thereof.

4. The compound of formula (I-i-1) according to claim 3, wherein Ar¹ is a phenyl group substituted with 1 or 2 groups selected from a halogen atom and a C₁ to C₅ alkoxy group, or a phenyl group, Y¹ is a nitrogen atom, Y² is a sulfur atom, R¹ is a hydrogen atom, a C₁ to C₅ alkyl group, an amino group or a C₁ to C₅ monoalkylamino group, R⁵ is a C₁ to C₆ alkyl group substituted at the terminal with 1 or 2 groups selected from "a phenyl group substituted with 1 or 2 groups selected from a halogen atom and a C₁ to C₅ alkoxy group, and a phenyl group", a C₃ to C₅ alkenyl group substituted at the terminal with 1 or 2 groups selected from "a phenyl group substituted with 1 or 2 groups selected from a halogen atom and a C₁ to C₅ alkoxy group, and a phenyl group", or a C₂ to C₇ alkoxycarbonyl group.

5. A thiazole derivative represented by formula (I-i-2): 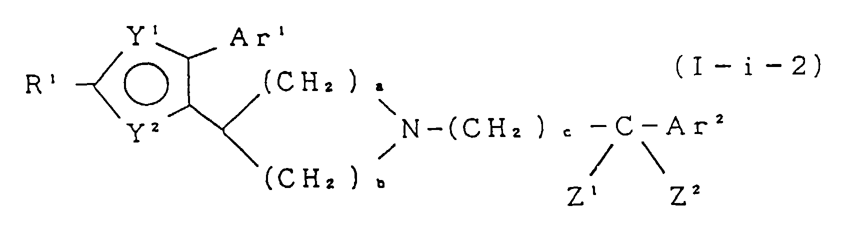 wherein Ar¹, Ar², Y¹, Y², R¹, a, b, c, Z¹ and Z² have the same meaning as in claim 1, or a pharmaceutically acceptable salt thereof.

6. The compound of formula (I-i-2) according to claim 5, wherein Ar¹ and Ar² are the same or different and each represents a phenyl group unsubstituted or substituted with 1 or 2 halogen atoms, Y¹ is a nitrogen atom, Y² is a sulfur atom, R¹ is a hydrogen atom, a C₁ to C₅ alkyl group, an amino group or a C₁ to C₅ monoalkylamino group, and Z¹ and Z² are combined to form an oxo group.

7. A thiazole derivative represented by formula (I-i-4): 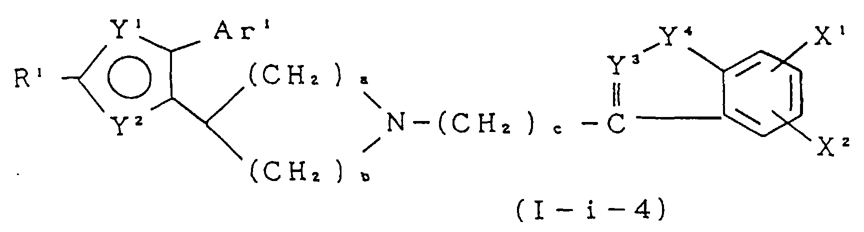 wherein Ar¹, Y¹, Y², Y³, Y⁴, R¹, a, b, c, X¹ and X² have the same meaning as in claim 1, or a pharmaceutically acceptable salt thereof.

8. The compound of formula (I-i-4) according to claim 7, wherein Ar¹ is a phenyl group unsubstituted or substituted with 1 or 2 halogen atoms, Y¹ is a nitrogen atom, Y² is a sulfur atom, and R¹ is a hydrogen atom, a C₁ to C₅ alkyl group, an amino group or a C₁ to C₅ monoalkylamino group.

9. A thiazole derivative represented by formula (I-ii): 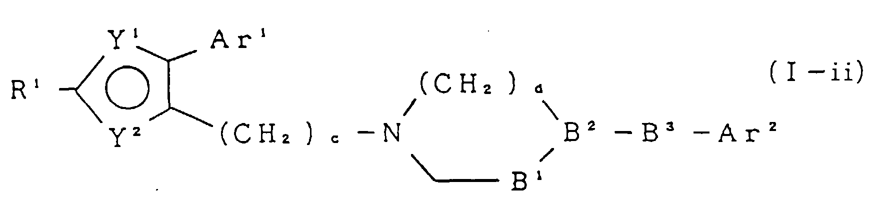 wherein Ar¹, Ar², Y¹, Y², R¹, c, d, B¹, B² and B³ have the same meaning as in claim 1, or a pharmaceutically acceptable salt thereof.

10. The compound of formula (I-ii) according to claim 9, wherein Ar¹ and Ar² are the same or different and each represents a phenyl group substituted with 1 or 2 groups selected arbitrarily from a halogen atom, a C₁ to C₅ alkyl group, a C₁ to C₅ alkoxy group, a hydroxyl group and a trifluoromethyl group, or a phenyl group, Y¹ is a nitrogen atom, Y² is a sulfur atom, and R¹ is a hydrogen atom, a C₁ to C₅ alkyl group, an amino group or a C₁ to C₅ monoalkylamino group.

11. The compound of formula (I-ii) according to claim 9, wherein Ar¹ and Ar² are the same or different and each represents a phenyl group substituted with 1 or 2 groups selected from a halogen atom and a C₁ to C₅ alkoxy group, or a phenyl group, Y¹ is a nitrogen atom, Y² is a sulfur atom, c is 2 or 3, B¹-B² is CH₂-CH, B³ is CO, and R¹ is a hydrogen atom, a methyl group, an amino group or a methylamino group.

12. A thiazole derivative represented by formula (I-iii): 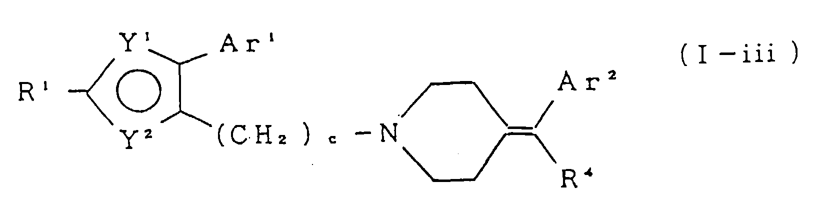 wherein Ar¹, Ar², Y¹, Y², R¹, R⁴ and c have the same meaning as in claim 1, or a pharmaceutically acceptable salt thereof.

13. The compound of formula (I-iii) according to claim 12, wherein Ar¹ and Ar² are the same or different and each represents a phenyl group substituted with 1 or 2 groups selected from a halogen atom, a C₁ to C₅ alkyl group, a C₁ to C₅ alkoxy group, a hydroxyl group and a trifluoromethyl group, or a phenyl group, Y¹ is a nitrogen atom, Y² is a sulfur atom, and R¹ is a hydrogen atom, a C₁ to C₅ alkyl group, an amino group or a C₁ to C₅ monoalkylamino group.

14. The compound of formula (I-iii) according to claim 12, wherein Ar¹ and Ar² are the same or different and each represents a phenyl group substituted with 1 or 2 groups selected from a halogen atom and a C₁ to C₅ alkoxy group, or a phenyl group,
Y¹ is a nitrogen atom, Y² is a sulfur atom, c is 2 or 3, R¹ is a hydrogen atom, a methyl group, an amino group or a methylamino group, and R⁴ is a hydrogen atom, a C₁ to C₅ alkyl group, a phenyl group substituted with a halogen atom, or a phenyl group.
